# EUROPEAN PATENT APPLICATION

(11) **EP 4 324 928 A1**
(43) Date of publication of application: **21.02.2024**
(21) Application number: 22191326.2
(22) Date of filing: 19.08.2022
(51) Int. Cl.: C12P 19/40, C12P 11/00, C12P 13/12

(54) **SYNTHETIC REAGENTS FOR ENZYME-CATALYSED ALKYLATION**

(71) Applicant: Universität Basel, 4001 Basel (CH)
(72) Inventor: The designation of the inventor has not yet been filed
(74) Representative: Schulz Junghans Patentanwälte PartGmbB

(57) **Abstract**

The present invention relates to an enzyme-catalyzed method for alkylating a substrate or a carrier compound using a sulfur-, or selenium-, or oxygen-based alkyl group donor.

## Description

### Field

The present invention relates to a method for alkylating a substrate or a carrier compound using a sulfur-, or selenium-, or oxygen-based alkyl group donor.

### Background

The methyl group is one of the most commonly occurring carbon fragments in low molecular weight therapeutics. During the development of drug candidates, methyl groups are commonly installed in an effort to improve potency and pharmacokinetic properties. For example, addition of a single methyl group in safinamide, a monoamine oxidase inhibitor used in the treatment of Parkinson's disease, increased potency by 20-fold and selectivity by nearly 1000-fold. Similar *magic methyl effects* have been observed in many examples and have inspired the emergence of late-stage methylation as a field of intense research.

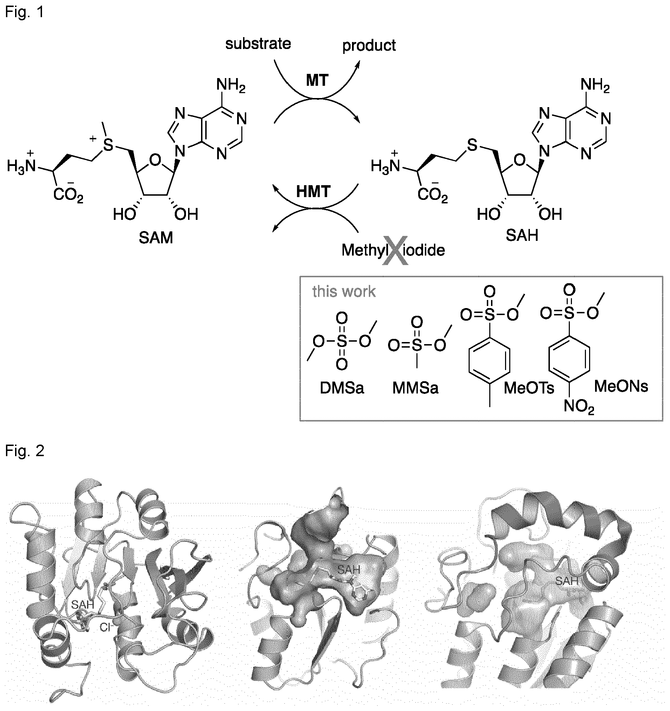

Although the current review literature on methylation strategies is largely dominated by developments in organic chemistry, biocatalysis may become an important alternative due to its potential for regio- and chemospecificity. Site-specific methylation of a vast array of substrates, from small molecules to proteins and nucleic acids, is a common transformation in biosynthetic pathways, signal transduction, epigenetics and restriction digestion. Consequently, methyltransferases (MTs) constitute a large enzyme family that covers a broad range of substrate specificities. The largest subgroup of this family is constituted by the *S-*adenosylmethionine (SAM, Figure 1) dependent MTs.

What makes MTs potentially highly attractive for preparative production is their remarkable ability to methylate complex molecules with high regio- and chemoselectivity. In the case of C-nucleophiles and thioethers, MTs can also mediate stereospecific methylation. MT-biocatalysis may not be limited to methylation. Due to their natural promiscuity, or as a result of introduced mutations, many MTs can also transfer larger alkyl chains such as ethyl-, propyl-, butyl-, isopropyl- or even methylcarboxy-groups or aromatic fragments. Despite this potential, MTs have rarely been used in biocatalysis so far. Currently, the major impediment to large-scale methylation is the cost of the stoichiometric co-substrate SAM in addition to its instability and poor atom economy as a methylation reagent. By contrast, NAD⁺/NADH-dependent oxidoreductases are routinely used for preparative transformations. In these reactions the nucleotide-based co-substrates NAD⁺ or NADH are regenerated using a sacrificial hydride donor or acceptor. The inventors and others surmised that MTs might be similarly successful in biocatalysis if SAM could be regenerated with similar efficiency. The inventors' current reaction uses a halide methyltransferase (HMT) that can methylate S-adenosylhomocysteine (SAH) with methyl iodide (Mel) to regenerate SAM. In combination with a secondary MT that consumes SAM to methylate its cognate substrate, HMT can recycle SAH 10³ times or more. This method with slight modification has been applied to the preparation of β-methyl-α-amino acids, fluoromethylated compounds, isotopically labelled compounds, N-alkylated pyrazoles, and indole alkaloids. Furthermore, directed evolution and profiling the catalytic promiscuity of naturally occurring HMT homologs was used to identify enzymes that generate SAM derivatives with larger alkyl groups.

In this current format, this SAM-regenerating system still suffers from a critical weakness. Methyl halides are toxic (Mel LD₅₀ rats: 76 mg/kg) and volatile (Mel boiling point: 42°C). In addition, the need to recover iodide from aqueous reaction media could add significant cost. Because of these problems, large-scale application of MT-biocatalysis may remain challenging, unless Mel can be replaced with a different reagent.

Based on the above-mentioned state of the art, the objective of the present invention is to provide means and methods to provide a non-toxic alkyl group donor for alkylation. This objective is attained by the subject-matter of the independent claims of the present specification, with further advantageous embodiments described in the dependent claims, examples, figures and general description of this specification.

### Summary of the Invention

In this report the inventors describe the ability of naturally occurring members of the thiopurine methyltransferase family to accept synthetic sulfate- and sulfonate-based methyl donors for the stereospecific S-methylation of SAH with remarkable efficiency. These findings allow to replace Mel with the non-volatile and less expensive methyl 4-toluenesulfonate (MeOTs) as a methyl donor in biocatalytic methylation reactions.

A first aspect of the invention relates to a method for production of an alkylated carrier compound comprising the steps:
a. providing an aqueous solution comprising a dealkylated carrier compound CC;
b. in an alkylation step applying:
   i. an alkyl-group donor X bound to an alkyl-group T; and
   ii. a methyltransferase transferring an alkyl group T from X to CC; yielding an alkylated carrier compound T-CC;
characterized in that the alkyl-group donor X is sulfur-, or selenium-, or oxygen-based.

A second aspect of the invention relates to a method for alkylation of a substrate, the method comprising the steps:
a. providing a substrate and a dealkylated carrier compound CC;
b. in an alkylation step, alkylating CC via the method according to any one of the preceding claims by transferring the alkyl group T yielding an alkylated carrier compound T-CC;
c. in an alkyl-substrate production step, transferring an alkyl group from T-CC to the substrate catalyzed by an N-, C-, O-, S-, or P-specific methyltransferase yielding an alkylated substrate and a dealkylated carrier compound;
wherein at least a part of the dealkylated carrier compound CC of step c is recycled to step b to regenerate the alkylated carrier compound T-CC.

A further aspect of the invention relates to a kit comprising a carrier compound as described above, a methyltransferase as described above, and a sulfur-, or oxygen- or, selenium-based alkyl-group donor as described above, and a carrier compound as described above.

A further aspect of the invention relates to a use of a carrier compound as described above, a methyltransferase as described above, a sulfur-, or oxygen- or, selenium-based alkyl - group donor as described above, and an N-, C-, O-, S-, or P-specific methyltransferase for production of an alkylated substrate.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

Any patent document cited herein shall be deemed incorporated by reference herein in its entirety.

### Sequences

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Using these algorithms mentioned above, it is possible to determine residues in equivalent positions as the reference sequence BxHMT (SEQ ID NO 1).

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

### Organic Chemistry

The term *alkyl* in the context of the present specification relates to a saturated linear, branched or (partially or completely) cyclic hydrocarbon. The term *unsubstituted Cₙ alkyl* when used herein in the narrowest sense relates to the moiety -CₙH₂ₙ- if used as a bridge between moieties of the molecule, or -CₙH₂ₙ₊₁ if used in the context of a terminal moiety. It may still contain fewer H atoms if a cyclical structure. Non-limiting examples for a C₁-C₄ alkyl are methyl, ethyl, propyl, n-butyl, 2-methylpropyl, *tert*-butyl, cyclo-butyl, cyclo-propyl, methyl-cyclo-propyl. Non-limiting examples for a C₁-C₆ alkyl include the examples given for C₁-C₄ alkyl above, and additionally n-pentyl, 2-methylbutyl, 3-methylbutyl, 1,1-dimethylpropyl, 1,2-dimethylpropyl, 1,2-dimethylpropyl, cyclo-pentyl, cyclo-hexyl, methyl-cyclo-pentyl. Non-limiting examples of a C₄-C₇ cycloalkyl moiety include cyclobutyl (-C₄H₇), cyclopentenyl (C₅H₉), and cyclohexenyl (C₆H₁₁) moieties. Where used in the context of chemical formulae, the following abbreviations may be used: *Me* is methyl CH₃, *Et* is ethyl -CH₂CH₃, *Prop* is propyl -(CH₂)₂CH₃ (n-propyl, n-pr) or -CH(CH₃)₂ (iso-propyl, i-pr), *but* is butyl -C₄H₉, - (CH₂)₃CH₃, -CHCH₃CH₂CH₃, -CH₂CH(CH₃)₂ or -C(CH₃)₃.

The term *alkene* in the context of the present specification relates to a hydrocarbon comprising a double bond. Unsubstituted alkene is of formula -CH=CH- when being located intramolecularly, and of formula -CH-CH₂ when being a terminal moiety.

The term *alkyne* in the context of the present specification relates to a hydrocarbon comprising a triple bond. Unsubstituted alkyne is of formula -CΞC- when being located intramolecularly, and of formula -CΞCH (-C₂H) when being a terminal moiety.

The term *aryl* in the context of the present specification relates to a cyclic aromatic C₅-C₁₀ hydrocarbon. Examples of aryl include, without being restricted to, phenyl and naphthyl.

The term *heteroaryl* in the context of the present specification relates to a cyclic aromatic C₂-C₉ hydrocarbon that comprises at least one heteroatom (e.g. N, O, S). Examples for heteroaryl include, without being restricted to, pyrrole, thiophene, furan, imidazole, pyrazole, thiazole, oxazole, pyridine, pyrimidine, thiazin, quinoline, benzofuran and indole.

An *alkyl aryl* in the context of the present specification relates to an alkyl group substituted by an aryl moiety. Particular examples are ethylphenyl, propylphenyl, butylphenyl and their higher homologues. An *alkyl heteroaryl* in the context of the present specification relates to an alkyl group substituted by a heteroaryl moiety.

The term *halogen-substituted group* refers to a group that is modified by one or several halogen atoms selected (independently) from F, Cl, Br, I.

The term *hydroxyl-substituted group* refers to a group that is modified by one or several hydroxyl groups OH.

The term *amino-substituted group* refers to a group that is modified by one or several amino groups NH₂.

The term *carboxyl-substituted group* refers to a group that is modified by one or several carboxyl groups COOH (also written as CO₂⁻).

The term *sulfonamide-substituted group* refers to a group that is modified by one or several sulfonamide groups -SO₂NHR or -NHSO₂R, or derivatives thereof, with R being defined further in the description.

The term *sulfate-substituted group* refers to a group of the chemical formula R-O-S(O₂)-O-R, with R being defined further in the description.

The term *sulfonate-substituted group* refers to a group of the chemical formula R-S(O₂)-O-R, with R being defined further in the description.

The term *sulfite-substituted group* refers to a group of the chemical formula R-O-S(O)-OR, with R being defined further in the description.

The term *sulfinate-substituted group* refers to a group of the chemical formula R-O-S(O)-R, with R being defined further in the description.

The term *thiosulfate-substituted group* refers to a group of the chemical formula R-S-S(O₂)-O-R, with R being defined further in the description.

The term *thiosulfonate-substituted group* refers to a group of the chemical formula R-S(O₂)-S-R, with R being defined further in the description.

The term *thiosulfinate-substituted group* refers to a group of the chemical formula R-S(O)-S-R, with R being defined further in the description.

The term *sultone* refers to a cyclic sulfonic ester. Two non-limiting examples are propane-1,3-sultone and 1,4-butane sultone.

The term *sulfamidate* refers to an amide derivative of a sulfate, wherein one oxygen is replaced by a nitrogen. In other words, a moiety where an oxygen atom and an amino group are bound to an SO₂ unit.

The term *thiirane*-1-oxide refers to ethylene episulfoxide.

The term *lactone* refers to a cyclic ester.

The term *thiolactone* refers to a cyclic thioester.

The term *sulfur-based* refers to a chemical moiety comprising at least one sulfur atom, wherein the chemical moiety is attached directly to the transferred alkyl group T.

The term *selenium-based* refers to a chemical moiety comprising at least one selenium atom, wherein the chemical moiety is attached directly to the transferred alkyl group T.

The term *oxygen-based* refers to a chemical moiety comprising a cyclic ester.

The term *amine-substituted group* refers to a group that is modified by one or several amine groups -NHR or -NR₂, or derivatives thereof, with each R being defined further in the description.

An *ester* refers to a group of -CO-O-R or -O-CO-R, with R being defined further in the description.

An *ether* refers to a group having one oxygen in between two saturated carbon atoms.

A *thioester* refers to a group of -S-CO-R or -CO-S-R, with R being defined further in the description.

A *thioether* refers to a group having one sulfur in between two saturated carbon atoms.

An *amide* refers to a group of -CONHR, with R being defined further in the description.

The term *nucleobase* in the context of the present specification relates to a purine or pyrimidine scaffold which may be chemically modified. Non-limiting examples of modification include methylation, deamination, hydroxylation and halogenation.

### Detailed Description of the Invention

A first aspect of the invention relates to a method for production of an alkylated carrier compound comprising the steps:
a. providing an aqueous solution comprising a dealkylated carrier compound CC;
b. in an alkylation step applying:
   i. T-X, wherein an alkyl-group donor X is bound to an alkyl-group T; and
   ii. a methyltransferase transferring an alkyl group T from X to CC; yielding an alkylated carrier compound T-CC;
characterized in that the alkyl-group donor X is sulfur-, or selenium-, or oxygen-based.

### Alkyl group T:

In certain embodiments, T is of the formula -CH₂-R⁰. In certain embodiments, T is of the formula -CHF-R⁰.

R⁰ is selected from the group comprising H, CN, F, Cl or an unsubstituted or substituted moiety MM being unfunctionalized or functionalized. MM is selected from the group comprising alkyl, aryl, heteroaryl, alkyl-aryl, alkyl-heteroaryl.

Functionalized MM comprises an alkene, an alkyne, an ether, a thioether, an ester, disulfide, and/or a thioester moiety.

If MM is functionalized, it is understood that the functional moiety may form including the -CH₂-R⁰ or -CHF-R⁰ moiety. This means that if MM is an alkyl functionalized with an ether moiety, T may be of formula -CH₂-O-CH₃. The same applies for an alkene, an alkyne, a thioether, an ester, disulfide, and a thioester moiety.

Substituted MM is substituted with one or two or three moieties selected from the group comprising halogen, OH, NH₂, N₃, CN, COOH, NO₂, CONH₂, SO₃⁻, SO₂NH₂, epoxide.

In certain embodiments, MM is selected from the group comprising alkyl, aryl, alkyl-aryl (unsubstituted or substituted, unfunctionalized or functionalized). In certain embodiments, MM is alkyl (unsubstituted or substituted, unfunctionalized or functionalized).

In certain embodiments, the alkyl moiety mentioned above is C₁-C₁₄ alkyl. In certain embodiments, the aryl moiety mentioned above is a monocyclic C₆ or bicyclic C₁₀ ring system. In certain embodiments, the heteroaryl moiety mentioned above is a monocyclic C₂-C₅ or bicyclic C₄-C₉ ring system. All these moieties may be unsubstituted or substituted, and unfunctionalized or functionalized.

In certain embodiments, the aryl group is selected from the group comprising benzene, naphthalene. In certain embodiments, the heteroaryl group is selected from the group comprising pyridine, pyrimidine, pyridazine, pyrazine, triazine, indole. In certain embodiments, alkyl is C₁-C₁₄ alkyl comprising 0, 1, 2, 3, 4, or 5 double bonds (alkene moieties) or comprising 0, 1, 2, or 3 triple bonds (alkyne moieties).

In certain embodiments, T-X is selected from the group comprising

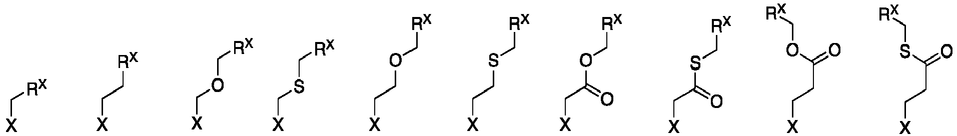

In certain embodiments, T-X is selected from the group comprising 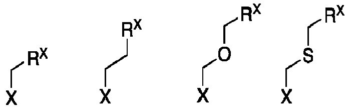
R^{X} is selected from the group comprising H, F, OCH₃, OCH₂CH₃, CN, -COOH, NO₂, CF₃, CHF₂, CH₂F, CH₂Cl, CH₂Br, CONH₂, CH₂CO₂R^{X1},CH₃, (CH₂)ₘCH₃, CH(CH₃)₂, C₂H, CH₂C₂H, CH=CH₂, CH₂CH=CH₂, unsubstituted or R^{X2}-substituted O-aryl or aryl, wherein O-aryl or aryl may be substituted with one or more R^{X2}. Each R^{X2} is independently selected from CH₃, (CH₂)ₘCH₃, CH(CH₃)₂, C(CH₃)₃, F, Cl, Br, I, OH, OR, NH₂, NHCH₃, NO₂, CN, CO₂, CF₃, CHF₂, CH₂F, CH₂Cl, CH₂Br, CONH₂, CH₂CO₂CH₃, SO₃⁻.
m is an integer selected from the group comprising 1, 2, 3, 4, 5, 6, 7, 8, 9, 10, 11, 12, 13, 14. R^{X1} is selected from the same group as R^{X} excluding CH₂CO2_{R}^{X1}. This means that R^{X1} is selected from H, F, OCH₃, OCH₂CH₃, CN, -COOH, NO₂, CF₃, CHF₂, CH₂F, CH₂Cl, CH₂Br, CONH₂, CH₃, (CH₂)ₘCH₃, CH(CH₃)₂, C₂H, CH₂C₂H, CH=CH₂, CH₂CH=CH₂, unsubstituted or R^{X2}-substituted O-aryl or aryl.

In certain embodiments, T-X is selected from the group comprising

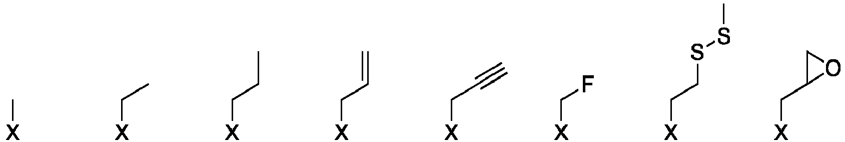

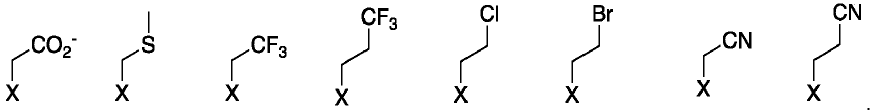

In certain embodiments, T is selected from the group comprising methyl, ethyl, propyl, propargyl, -CH₂F. In certain embodiments, T is methyl.

### Carrier compound CC:

In certain embodiments, CC is of the general formula: Z-P-Rib-Nuc,
wherein
   - Z is of the formula -CH₂-R^{Z} with R^{Z} being selected from H, or an unsubstituted or substituted moiety NN being unfunctionalized or functionalized, wherein NN is selected from the group comprising alkyl, aryl, heteroaryl, alkyl-aryl, alkyl-heteroaryl,
      wherein functionalized NN comprises an alkene, an alkyne, an ether, a thioether, an ester, and/or a thioester moiety,
      wherein substituted NN is substituted with one or two or three moieties selected from the group comprising halogen, CN, COOH, NO₂, NH₃, CONH₂, SO₃H;
      particularly NN is alkyl (unsubstituted or substituted, unfunctionalized or functionalized);
   - P is a sulfur atom or a selenium atom;
   - Rib is selected from the group comprising ribose, deoxyribose, 2-O-methyl ribose, carbaribose, 4-thioribose, particularly Rib is ribose;
   - Nuc is a naturally occurring nucleobase or a heteroaromatic mono- or bicyclic synthetic nucleobase derivative (methylated, deaminated, hydroxylated and/or halogenated nucleobase);
wherein P is connected to the 5' C atom of Rib, and Nuc is connected to the O atom of the 1' C atom of Rib.

Formula of Z-P-Rib-Nuc: 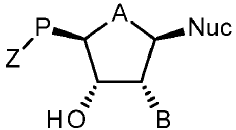 with A being selected from O, S and CH₂, and B being selected from the group comprising H and OH, -OCH₃.
Ribose: 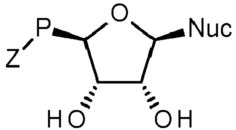
Deoxyribose: 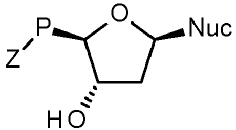
2-O-methyl ribose: 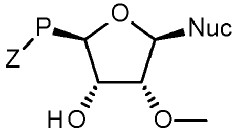
Carbaribose: 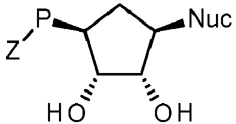
4-thioribose:

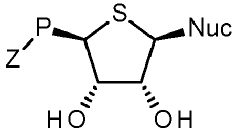

In certain embodiments, CC is selected from the group comprising 5'-deoxy-5'-alkylthionucleoside and 5'-deoxy-5'-alkylselenonucleoside, with alkyl being selected from the group comprising methyl, fluormethyl, ethyl, propyl, 2-carboxy-2-aminoethyl, 3-aminopropyl, 3-hydroxypropyl, 3-carboxypropyl, 4-carboxybutyl, 4-aminobutyl, 4-hydroxybutyl, 3-carboxy-3-aminopropyl, 4-carboxy-4-aminobutyl. In certain embodiments, alkyl is selected from the group comprising 2-carboxy-2-aminoethyl, 3-carboxy-3-aminopropyl.

In certain embodiments, CC is selected from the group comprising S-nucleosyl-homocysteine, Se-nucleosyl-homoselenocysteine, S-inosyl-L-homocysteine, Se-inosyl-L-homoselenocysteine, S-7-deazaadenosyl-L-homocysteine, S-7-deazaadenosyl-L-homoselenocysteine, S-formycinyl-L-homocysteine, Se-formycinyl-L-homoselenocysteine, S-aristeromycyl-L-homocysteine, Se- aristeromycyl-L-homoselenocysteine.

In certain embodiments, CC is selected from the group comprising the formulas of the following table:

| Systematic Name | trivial name | structure |
|---|---|---|
| (S)-2-amino-4-((((2S,3S,4R,5R)-5-(6-amino-9H-purin-9-yl)-3,4-dihydroxytetrahydrofuran-2-yl)methyl)thio)-1-(λ¹-oxidaneyl)butan-1-one | S-adenosyl-L-homocysteine | 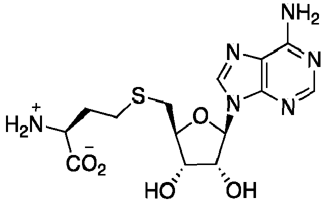 |
| (*R*)-4-((((2*S*,3*S*,4*R*,5*R*)-5-(6-amino-9*H*-purin-9-yl)-3,4-dihydroxytetrahydrofuran-2-yl)methyl)thio)-2-hydroxybutanoate | | 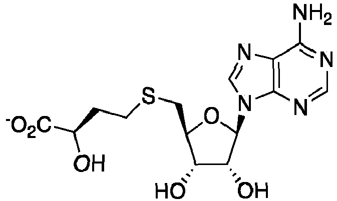 |
| (*S*)-4-((((2*S*,3*S*,4*R*,5*R*)-5-(4-amino-7*H*-pyrrolo[2,3-*d*]pyrimidin-7-yl)-3,4-dihydroxytetrahydrofuran-2-yl)methyl)thio)-2-ammoniobutanoate | S-7-deazaadenosyl-L-homocysteine | 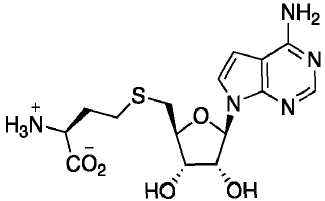 |
| (*S*)-2-ammonio-4-((((2S,3S,4R,5R)-3,4-dihydroxy-5-(6-hydroxy-9*H-*purin-9-yl)tetrahydrofuran-2-yl)methyl)thio)butanoate | S-inosyl-L-homocysteine | 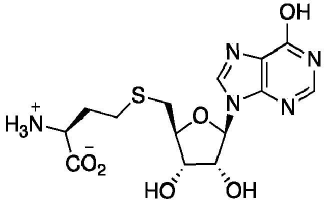 |
| (*S*)-2-ammonio-4-((((2S,3S,4R,5R)-3,4-dihydroxy-5-(9*H*-purin-9-yl)tetrahydrofuran-2-yl)methyl)thio)butanoate | S-nebularinyl-L-homocysteine | 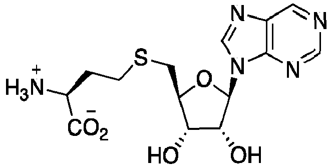 |
| (S)-4-((((2S,3S,4R,5S)-5-((R)-7-amino-3*H*-pyrazolo[4,3-*d*]pyrimidin-3-yl)-3,4-dihydroxytetrahydrofuran-2-yl)methyl)thio)-2-ammoniobutanoate | S-formycinyl-L-homocysteine | 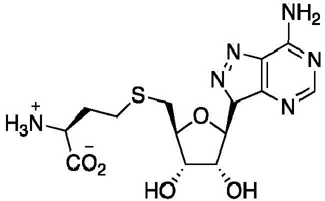 |
| (S)-4-((((1S,2R,3S,4R)-4-(6-amino-9H-purin-9-yl)-2,3-dihydroxycyclopentyl)methyl)th io)-2-ammoniobutanoate | S- aristeromycyl-L-homocysteine | 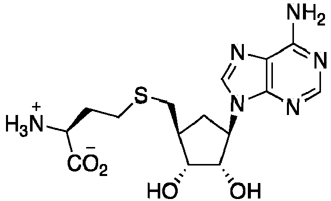 |
| (S)-4-((((2S,3S,4R,5R)-5-(4-amino-1*H*-imidazo[4,5-c]pyridin-1-yl)-3,4-dihydroxytetrahydrofuran-2-yl)methyl)thio)-2-ammoniobutanoate | S-(3-deazaadenosyl)-L-homocysteine | 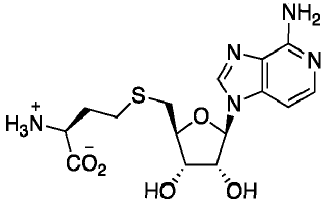 |
| (S)-4-((((2S,3S,5R)-5-(6-amino-9*H*-purin-9-yl)-3-hydroxytetrahydrofuran-2-yl)methyl)thio)-2-ammoniobutanoate | S-2-deoxy-adenosine-L-homocysteine | 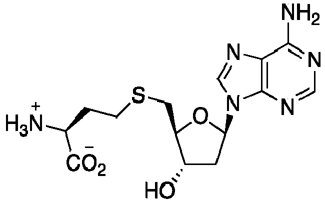 |
| (S)-4-((((2S,3S,4R,5R)-5-(4-amino-2-oxopyrimidin-1(2*H*)-yl)-3,4-dihydroxytetrahydrofuran-2-yl)methyl)thio)-2-ammoniobutanoate | S-cytidyl-L-homocysteine | 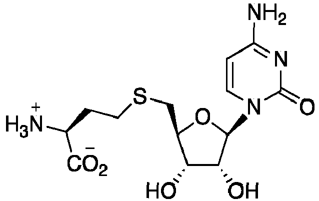 |
| (R)-3-((((2S,3S,4R,5R)-5-(6-amino-9*H*-purin-9-yl)-3,4-dihydroxytetrahydrofuran-2-yl)methyl)thio)-2-ammoniopropanoate | S-adenosyl-L-cysteine | 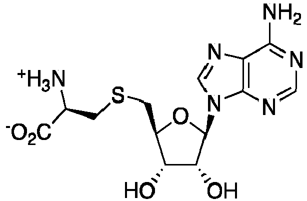 |
| 3-((((2*S*,3*S*,4*R*,5*R*)-5-(6-amino-9*H*-purin-9-yl)-3,4-dihydroxytetrahydrofuran-2-yl)methyl)thio)propan-1-aminium | | 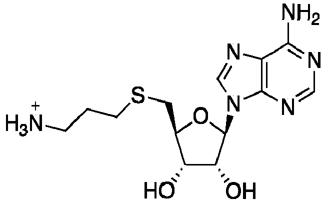 |
| (2R,3R,4S,5S)-2-(6-amino-9H-purin-9-yl)-5-(((3-hydroxypropyl)thio)methyl)tetra hydrofuran-3,4-diol | | 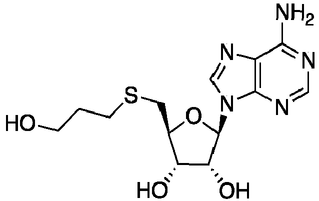 |
| 4-((((2*S*,3*S*,4*R*,5*R*)-5-(6-amino-9*H*-purin-9-yl)-3,4-dihydroxytetrahydrofuran-2-yl)methyl)thio)butanoate | | 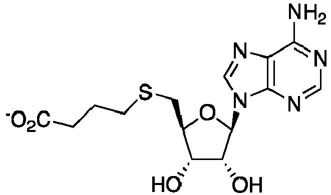 |
| (*S*)-4-((((2*S*,3*S*,4*R*,5*R*)-5-(6-amino-9*H*-purin-9-yl)-3-hydroxy-4-methoxytetrahydrofuran-2-yl)methyl)thio)-2-ammoniobutanoate | | 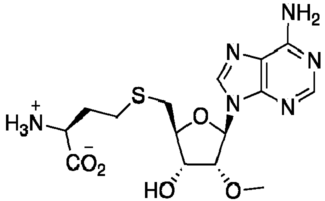 |
| (2*R*,3*R*,4*S*,5*S*)-2-(6-amino-9*H-*purin-9-yl)-5-((methylthio)methyl)tetrahydrof uran-3,4-diol | 5'-Methylthioadenos ine | 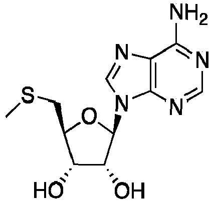 |
| (2*R*,3*R*,4*S*,5*S*)-2-(6-amino-9*H-*purin-9-yl)-5-((ethylthio)methyl)tetrahydrofur an-3,4-diol | 5'-Ethylthioadenosin e | 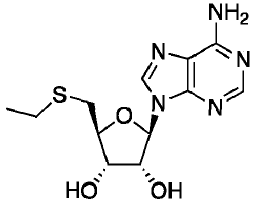 |
| (2*R*,3*R*,4*S*,5*S*)-2-(6-amino-9*H-*purin-9-yl)-5-((propylthio)methyl)tetrahydrof uran-3,4-diol | 5'-Propylthioadenosi ne | 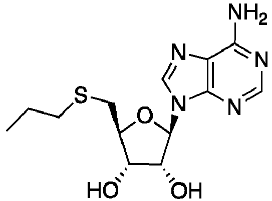 |
| 5-((((2*S*,3*S*,4*R*,5*R*)-5-(6-amino-9*H*-purin-9-yl)-3,4-dihydroxytetrahydrofuran-2-yl)methyl)thio)pentanoate | | 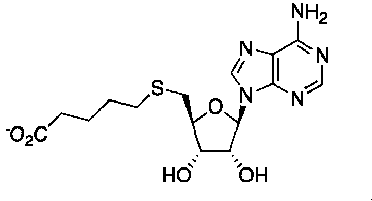 |
| (*S*)-4-((((2*S*,3*S*,4*R*,5*R*)-5-(6-amino-9*H*-purin-9-yl)-3,4-dihydroxytetrahydrofuran-2-yl)methyl) selanyl)-2-ammoniobutanoate | S-adenosyl-L-hom oselenocystei ne | 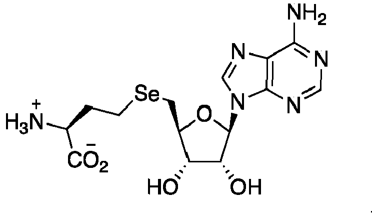 |
| (*R*)-4-((((2*S*,3*S*,4*R*,5*R*)-5-(6-amino-9*H*-purin-9-yl)-3,4-dihydroxytetrahydrofuran-2-yl)methyl) selanyl)-2-hydroxybutanoate | | 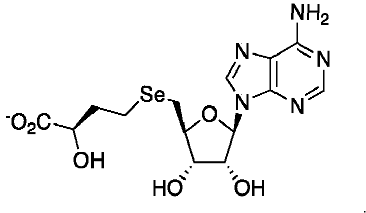 |
| (*S*)-4-((((1*S*,2*R*,3*S*,4*R*)-4-(6-amino-9*H*-purin-9-yl)-2,3-d ihydroxycyclopentyl) methyl) s elanyl)-2-ammoniobutanoate | S-7-deazaadenosyl-L-hom oselenocystei ne | 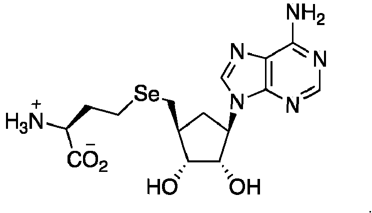 |
| (*S*)-2-ammonio-4-((((2*S*,3*S*,4*R*,5*R*)-3,4-dihydroxy-5-(6-hydroxy-9*H-*purin-9-yl)tetrahydrofuran-2-yl)methyl)selanyl)butanoate | S-inosyl-L-homoselenocystei ne | 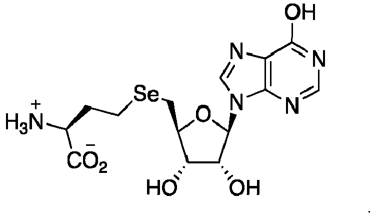 |
| (*S*)-2-ammonio-4-((((2*S*,3*S*,4*R*,5*R*)-3,4-dihydroxy-5-(9*H*-purin-9- | S-nebularinyl-L-homoselenocystei ne | 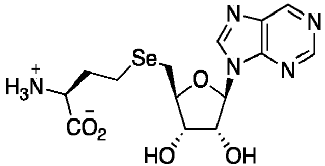 |
| yl)tetrahydrofuran-2-yl)methyl)selanyl)butanoate | | |
| (*S*)-4-((((2*S*,3*S*,4*R*,5*S*)-5-((*R*)-7-amino-3*H*-pyrazolo[4,3-*d*]pyrimidin-3-yl)-3,4-dihydroxytetrahydrofuran-2-yl)methyl) selanyl)-2-ammoniobutanoate | S-formycinyl-L-homoselenocystei ne | 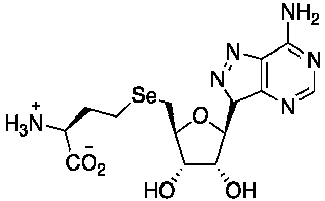 |
| (*S*)-4-((((1*S*,2*R*,3*S*,4*R*)-4-(6-amino-9*H*-purin-9-yl)-2,3-d ihydroxycyclopentyl) methyl) s elanyl)-2-ammoniobutanoate | S- aristeromycyl-L-hom oselenocystei ne | 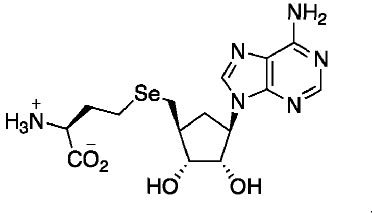 |
| (S)-4-((((2S,3S,4R,5R)-5-(4-amino-1*H*-imidazo[4,5-c]pyridin-1-yl)-3,4-dihydroxytetrahydrofuran-2-yl)methyl)selanyl)-2-ammoniobutanoate | S-(3-deazaadenosyl)-L-homoselenocystei ne | 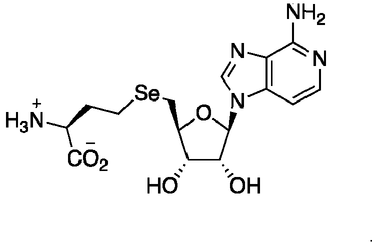 |
| (S)-4-((((2S,3S,5R)-5-(6-amino-9*H*-purin-9-yl)-3-hydroxytetrahydrofuran-2-yl)methyl)selanyl)-2-ammoniobutanoate | S-2-deoxy-adenosine-L-homoselenocystei ne | 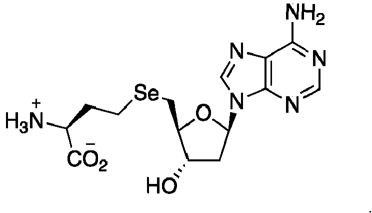 |
| (S)-4-((((2S,3S,4R,5R)-5-(4-amino-2-oxopyrimidin-1(2*H*)-yl)-3,4-dihydroxytetrahydrofuran-2-yl)methyl) selanyl)-2-ammoniobutanoate | S-cytidyl-L-homoselenocystei ne | 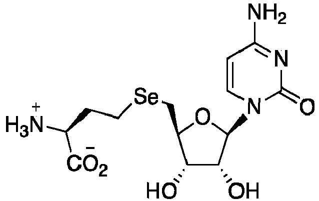 |
| (*R*)-3-((((2*S*,3*S*,4*R*,5*R*)-5-(6-amino-9*H*-purin-9-yl)-3,4-dihydroxytetrahydrofuran-2-yl)methyl) selanyl)-2-ammoniopropanoate | S-adenosyl-L-selenocysteine | 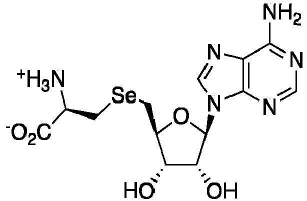 |
| 3-((((2*S*,3*S*,4*R*,5*R*)-5-(6-amino-9*H*-purin-9-yl)-3,4-dihydroxytetrahydrofuran-2-yl)methyl)selanyl)propan-1-aminium | | 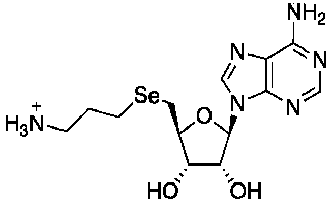 |
| (2*R*,3*R*,4*S*,5*S*)-2-(6-amino-9*H-*purin-9-yl)-5-(((3-hydroxypropyl)selanyl)methyl)t etrahydrofuran-3,4-diol | | 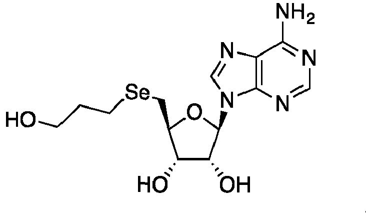 |
| 4-((((2*S*,3*S*,4*R*,5*R*)-5-(6-amino-9*H*-purin-9-yl)-3,4-dihydroxytetrahydrofuran-2-yl)methyl)selanyl)butanoate | | 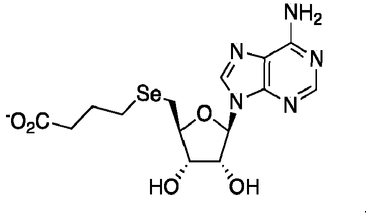 |
| (S)-4-((((2S,3S,4R,5R)-5-(6-amino-9*H*-purin-9-yl)-3-hydroxy-4-methoxytetrahydrofuran-2-yl)methyl) selanyl)-2-ammoniobutanoate | | 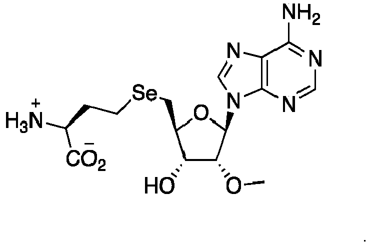 |
| (2R,3R,4S,5S)-2-(6-amino-9H-purin-9-yl)-5-((methylselanyl)methyl)tetrahy drofuran-3,4-diol | 5'-Methylselenoade nosine | 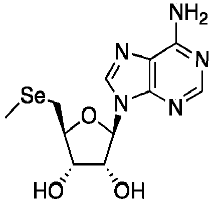 |
| (2R,3R,4S,5S)-2-(6-amino-9H-purin-9-yl)-5-((ethylselanyl)methyl)tetrahydr ofuran-3,4-diol | 5'-Ethylselenoadeno sine | 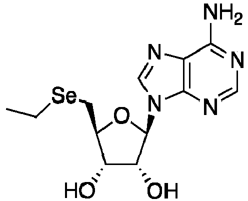 |
| (2R,3R,4S,5S)-2-(6-amino-9H-purin-9-yl)-5-((propylselanyl)methyl)tetrahyd rofuran-3,4-diol | 5'-Propylselenoaden osine | 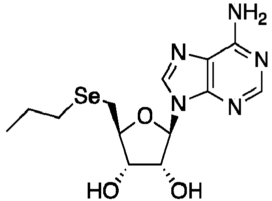 |
| 5-((((2S,3S,4R,5R)-5-(6-amino-9*H*-purin-9-yl)-3,4-dihydroxytetrahydrofuran-2-yl)methyl)selanyl)pentanoate | | 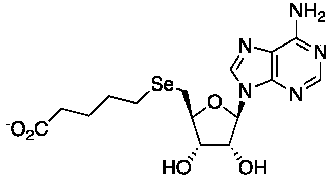 |

In certain embodiments, CC is selected from the group comprising the formulas of the following table:

| Systematic Name |
|---|
| (S)-2-amino-4-((((2S,3S,4R,5R)-5-(6-amino-9H-purin-9-yl)-3,4-dihydroxytetrahydrofuran-2-yl)methyl)thio)-1-(λ¹-oxidaneyl)butan-1-one |
| (*R*)-4-((((2*S*,3*S*,4*R*,5*R*)-5-(6-amino-9*H*-purin-9-yl)-3,4-dihydroxytetrahydrofuran-2-yl)methyl)thio)-2-hyd roxybutanoate |
| (*S*)-4-((((2*S*,3*S*,4*R*,5*R*)-5-(4-amino-7*H*-pyrrolo[2,3-*d*]pyrimidin-7-yl)-3,4-dihydroxytetrahydrofuran-2-yl)methyl)thio)-2-ammoniobutanoate |
| (*S*)-2-ammonio-4-((((2*S*,3*S*,4*R*,5*R*)-3,4-dihydroxy-5-(6-hydroxy-9*H*-purin-9-yl)tetrahydrofuran-2-yl)methyl)thio)butanoate |
| (*S*)-2-ammonio-4-((((2*S*,3*S*,4*R*,5*R*)-3,4-dihydroxy-5-(9*H*-purin-9-yl)tetrahydrofuran-2-yl)methyl)thio)butanoate |
| (*S*)-4-((((2*S*,3*S*,4*R*,5*S*)-5-((*R*)-7-amino-3*H*-pyrazolo[4,3-*d*]pyrimidin-3-yl)-3,4-dihydroxytetrahydrofuran-2-yl)methyl)thio)-2-ammoniobutanoate |
| (S)-4-((((1S,2R,3S,4R)-4-(6-amino-9H-purin-9-yl)-2,3-dihydroxycyclopentyl)methyl)thio)-2-ammoniobutanoate |
| (*S*)-4-((((2*S*,3*S*,4*R*,5*R*)-5-(4-amino-1*H*-imidazo[4,5-*c*]pyridin-1-yl)-3,4-dihydroxytetrahydrofuran-2-yl)methyl)thio)-2-ammoniobutanoate |
| (*S*)-4-((((2*S*,3*S*,5*R*)-5-(6-amino-9*H*-purin-9-yl)-3-hydroxytetrahydrofuran-2-yl)methyl)thio)-2-ammoniobutanoate |
| (*S*)-4-((((2*S*,3*S*,4*R*,5*R*)-5-(4-amino-2-oxopyrimidin-1(2*H*)-yl)-3,4-dihydroxytetrahydrofuran-2-yl)methyl)thio)-2-ammoniobutanoate |
| (*R*)-3-((((2*S*,3*S*,4*R*,5*R*)-5-(6-amino-9*H*-purin-9-yl)-3,4-dihydroxytetrahydrofuran-2-yl)methyl)thio)-2-ammoniopropanoate |
| 3-((((2*S*,3*S*,4*R*,5*R*)-5-(6-amino-9*H*-purin-9-yl)-3,4-dihydroxytetrahydrofuran-2-yl)methyl)thio)propan-1-aminium |
| (2*R*,3*R*,4*S*,5*S*)-2-(6-amino-9*H*-purin-9-yl)-5-(((3-hydroxypropyl)thio)methyl)tetrahydrofuran-3,4-diol |
| 4-((((2*S*,3*S*,4*R*,5*R*)-5-(6-amino-9*H*-purin-9-yl)-3,4-dihydroxytetrahydrofuran-2-yl)methyl)thio)butanoate |
| (*S*)-4-((((2*S*,3*S*,4*R*,5*R*)-5-(6-amino-9*H*-purin-9-yl)-3-hydroxy-4-methoxytetrahydrofuran-2-yl)methyl)thio)-2-ammoniobutanoate |
| (2*R*,3*R*,4*S*,5*S*)-2-(6-amino-9*H*-purin-9-yl)-5-((methylthio)methyl)tetrahydrofuran-3,4-diol |
| (2*R*,3*R*,4*S*,5*S*)-2-(6-amino-9*H*-purin-9-yl)-5-((ethylthio)methyl)tetrahydrofuran-3,4-diol |
| (2*R*,3*R*,4*S*,5*S*)-2-(6-amino-9*H*-purin-9-yl)-5-((propylthio)methyl)tetrahydrofuran-3,4-diol |

In certain embodiments, CC is S-adenosyl-L-homocysteine (SAH).

### Alkyl group donor X:

In certain embodiments, X comprises a moiety selected from the group comprising sulfate, sulfonate, sulfite, sulfinate, thiosulfate, thiosulfonate, thiosulfinate, wherein T is attached to said moiety.

Formulas: Sulfate: 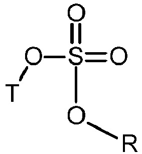 ; Sulfonate: 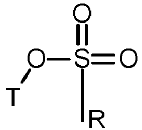 ; Sulfite: 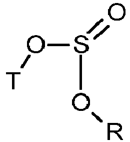 Sulfinate: 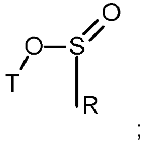 Thiosulfate: 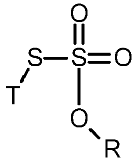 ;Thiosulfonate: 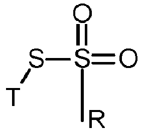 Thiosulfinate:

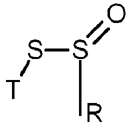

In certain embodiments, X is selected from the group comprising alkylsulfate, arylsulfate, heteroarylsulfate, alkylsulfonate, arylsulfonate, heteroarylsulfonate, alkylsulfite, arylsulfite, heteroarylsulfite, alkylsulfinate, arylsulfinate, heteroarylsulfinate, alkylthiosulfate, arylthiosulfate, heteroarylthiosulfate, alkylthiosulfonate, arylthiosulfonate, heteroarylthiosulfonate, alkylthiosulfinate, arylthiosulfinate, heteroarylthiosulfinate.

In certain embodiments, the aryl group is a substituted or unsubstituted monocyclic or bicyclic compound. In certain embodiments, the aryl group is selected from the group comprising benzene, naphthalene.

In certain embodiments, the heteroaryl group is a substituted or unsubstituted monocyclic or bicyclic compound. In certain embodiments, the heteroaryl group is selected from the group comprising pyridine, pyrimidine, pyridazine, pyrazine, triazine, indole.

In certain embodiments, the alkyl group is a substituted or unsubstituted linear or cyclic C₁-C₁₅ hydrocarbon.

In certain embodiments, T-X is selected from the group comprising 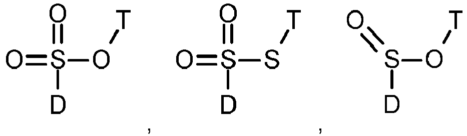 with D being selected from -R^{D1}, -OR^{D2}, -SR^{D2}, -NR^{D2}R^{D3}, with
- R^{D1} being selected from the group comprising an unsubstituted or substituted moiety LL being unfunctionalized or functionalized, wherein LL is selected from the group comprising alkyl, aryl, heteroaryl, alkyl-aryl, alkyl-heteroaryl, wherein functionalized LL comprises an alkene, an alkyne, an ether, a thioether, an ester, and/or a thioester moiety, wherein substituted LL is substituted with one or two or three moieties selected from the group comprising halogen, CN, COOH, NO₂, NH₃, CONH₂, SO₃H;

- R^{D2} and R^{D3} being independently selected from the group comprising H or R^{D1}.

In certain embodiments, T-X is selected from the group comprising 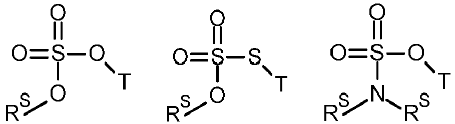 with each R^{S} being independently selected from the group comprising H, CH₃, (CH₂)ₕCH₃, CH(CH₃)₂, C(CH₃)₃, CN, CF₃, CHF₂, CH₂F, CH₂Cl, CH₂Br, CH₂NO₂, CH₂CO₂CH₃, C₆H₁₁.

In certain embodiments, T-X is selected from the group comprising 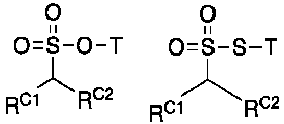 with R^{C1} and R^{C2} being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂-, CF₃, CHF₂, CH₂F, CH₂Cl, CH₂Br, CONH₂, CH₂CO₂CH₃, C₆H₁₁, SO₃-.

In certain embodiments, T-X is selected from the group comprising 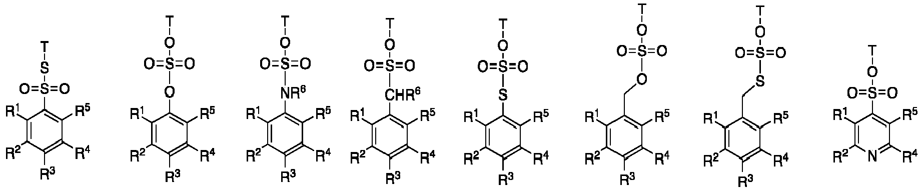 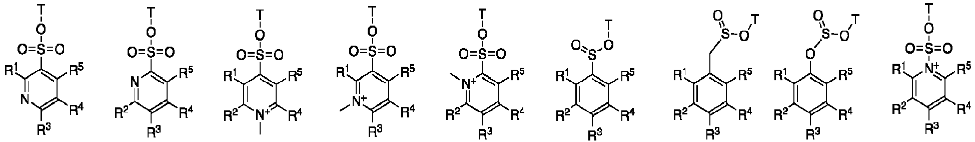 with R¹ - R⁵ being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂, CF₃, CHF₂, CH₂F, CH₂Cl, CH₂Br, CONH₂, CH₂CO₂CH₃, S(CH₃)₂⁺, N(CH₃)₃⁺, SO₃⁻;

In certain embodiments, T-X is selected from the group comprising R¹ - R⁵ are independently selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃, more particularly from H, CH₃, F, Cl, Br, OMe, CF₃, NO₂, CN.

Each R⁶ is independently selected from the group comprising H, CH_{3.} h is an integer selected from the group comprising 1, 2, 3, 4, 5, 6, 7, 8, 9, 10, 11, 12, 13, 14.

In certain embodiments, T-X is selected from the group comprising 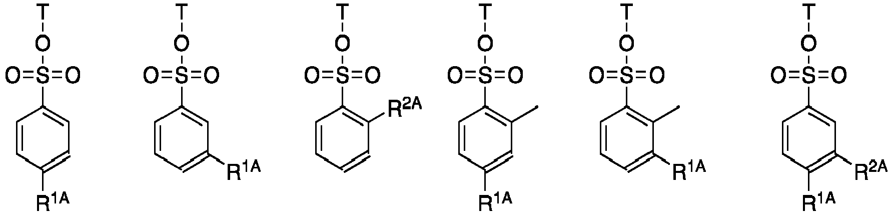 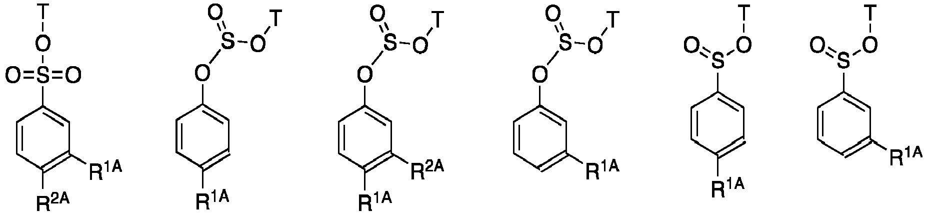
with R^{1A} being selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃,
with R^{2A} being selected from the group comprising H, CH₃, F, Cl, Br, OMe, CF₃, NO₂, CN.

In certain embodiments, T-X is 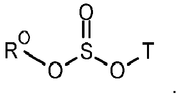 R^{O} being selected from the group comprising H, CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃, C₆H₁₁.

In certain embodiments, T-X is

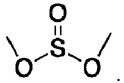

In certain embodiments, T-X is 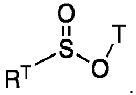 R^{T} is selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁.

In certain embodiments, T-X is 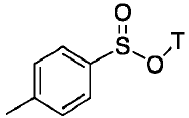 k is an integer selected from the group comprising 1, 2, 3, 4, 5, 6, 7, 8, 9, 10, 11, 12, 13, 14.

In certain embodiments, X is sulfur-based.

In certain embodiments, X comprises a sulfonate or a sulfate moiety.

In certain embodiments, T-X is selected from the group comprising methyl toluenesulfonate (MeOTs), methyl 4-nitrobenzenesulfonate (MeONs), dimethyl sulfate (DMSa), or methyl methanesulfonate (MMSa), dimethyl sulfite, S-methyl thiosulfate. In certain embodiments, the alkyl-group donor is MeOTs.

### Cyclic alkyl group donor T-X:

In certain embodiments, T-X is selected from the group comprising a cyclic sulfate, a cyclic sulfite, a sultone, a cyclic sulfamidate, a thiirane-1-oxide, a lactone or a thiolactone.

In certain embodiments, T-X is selected from the group comprising

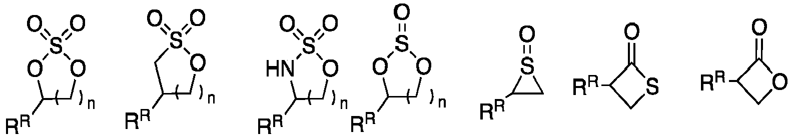

In certain embodiments, T-X is selected from the group comprising 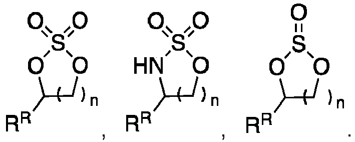 In certain embodiments, T-X is 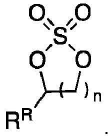 R^{R} is selected from the group comprising H, OR, NO₂, CN, COOH, CF₃, CHF₂, CH₂F, CH₂Cl, CH₂Br, CONH₂, CH₂CO₂Me, CH₃, (CH₂)ₙCH₃, with n being an integer selected from the group comprising 1, 2, 3, 4, 5, 6, 7, 8, 9, 10, 11, 12, 13, 14.

### Selenium-based X:

In certain embodiments, X comprises a moiety selected from the group comprising selenite, selenate, selenonate, seleninate. Selenate: 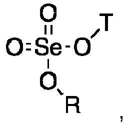 Selenite: 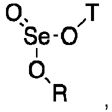 Selenonate: 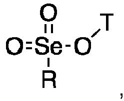 Seleninate:

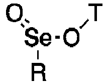

In certain embodiments, T-X is selected from the group comprising 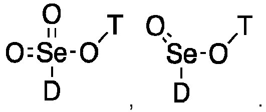 with D being selected from -R^{D1}, -OR^{D2}, -SR^{D2}, -NR^{D2}R^{D3}, with
- R^{D1} being selected from the group comprising an unsubstituted or substituted moiety LL being unfunctionalized or functionalized, wherein LL is selected from the group comprising alkyl, aryl, heteroaryl, alkyl-aryl, alkyl-heteroaryl, wherein functionalized LL comprises an alkene, an alkyne, an ether, a thioether, an ester, and/or a thioester moiety,
   wherein substituted LL is substituted with one or two or three moieties selected from the group comprising halogen, CN, COOH, NO₂, NH₃, CONH₂, SO₃H;
- R^{D2} and R^{D3} being independently selected from the group comprising H or R^{D1}.

In certain embodiments, T-X is selected from the group comprising
a. 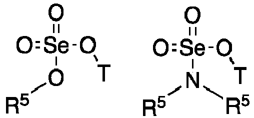 with each R^{s} being independently selected from the group comprising H, CH₃, (CH₂)ₕCH₃, CH(CH ₃)₂, C(CH₃)₃, CN, CF₃, CHF₂, CH₂F, CH₂Cl, CH₂Br, CH₂NO₂, CH₂CO₂CH₃, C₆H₁₁;
b. 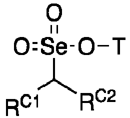 with R^{C1} and R^{C2} being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂₋, CF₃, CHF₂, CH₂F, CH₂Cl, CH₂Br, CONH₂, CH₂CO₂CH₃, C₆H₁₁, SO₃⁻
c. 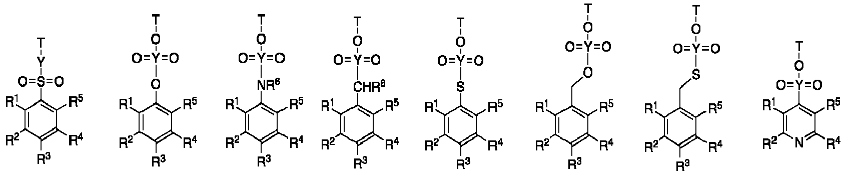 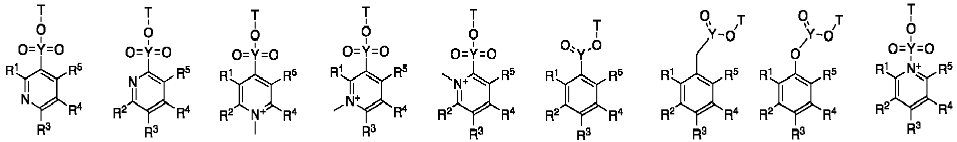
   With Y = Se; with R¹ - R⁵ being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂, CF₃, CHF₂, CH₂F, CH₂Cl, CH₂Br, CONH₂, CH₂CO₂CH₃, S(CH₃)₂⁺Cl, N(CH₃)₃⁺Cl, SO₃Na
   in particular from H, CH₃, F, Cl, Br, OMe, CF₃, NO₂, CN, CO₂Me, CH(CH₃)₂, S(CH₃)₂⁺Cl, N(CH₃)₃⁺Cl, C(CH₃)₃, more particularly from H, CH₃, F, Cl, Br, OMe, CF₃, NO₂, CN,
   with each R⁶ being independently selected from the group comprising H, CH₃;
   with h being an integer selected from the group comprising 1, 2, 3, 4, 5, 6, 7, 8, 9, 10, 11, 12, 13, 14.

In certain embodiments, T-X is selected from the group comprising 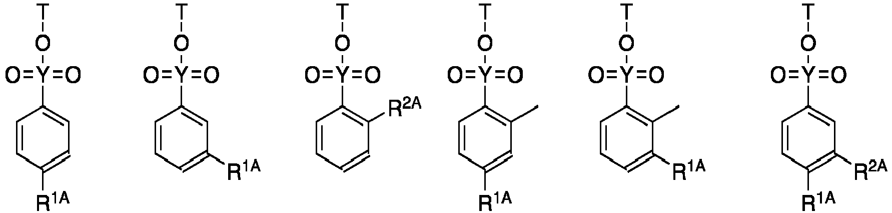 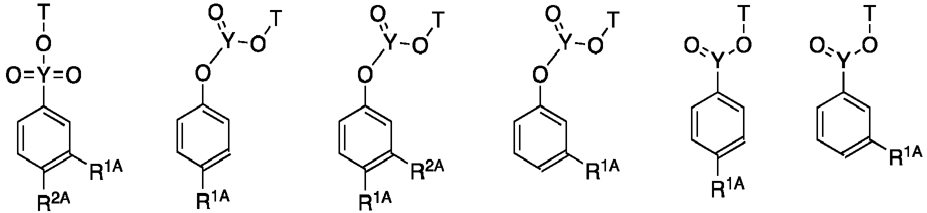
With Y = Se; with R^{1A} being selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃,
with R^{2A} being selected from the group comprising H, CH₃, F, Cl, Br, OMe, CF₃, NO₂, CN, 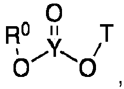 particularly: 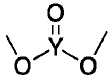
With Y = Se; with R^{O} being selected from the group comprising H, CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃, C₆H₁₁; 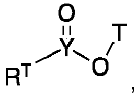 particularly 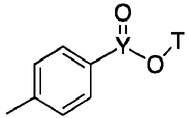
With Y = Se; with R^{T} being selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁
with k being an integer selected from the group comprising1, 2, 3, 4, 5, 6, 7, 8, 9, 10, 11, 12, 13, 14.

In certain embodiments, T-X is selected from the group comprising 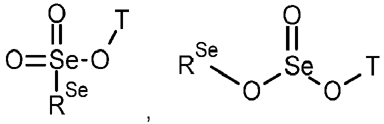 particularly wherein T-X is selected from the group comprising 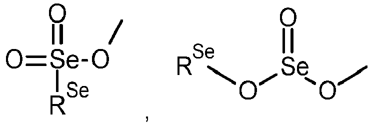 wherein
R^{Se} is selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁, with k being an integer selected from the group comprising 1 - 14,
particularly R^{Se} is CH_{3.}

In certain embodiments, T-X is selected from the group comprising 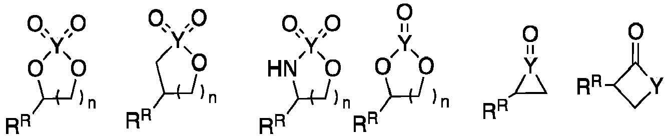 with Y being Se.

### Methyltransferase:

In certain embodiments, the methyltransferase is derived from or is an S-adenosylmethionine (SAM)-dependent methyltransferase.

In certain embodiments, the SAM-dependent methyltransferase is capable of methylating anionic nucleophiles, particularly anionic nucleophiles selected from the group comprising halides, phosphates, sulfates, sulfonates, sulfites, thiolates, sulfinic acids, carboxylates, N-oxides, cyanate, thiocyanate.

In certain embodiments, the methyltransferase belongs to a protein family (PF) or is derived from a protein belonging to a PF, wherein the PF is selected from the group comprising PF05724 (including thiopurine methyltransferases, halide methyltransferases and N-oxide methyltransferases), PF03492 (including carboxyl methyltransferases), PF08241 (including thiol S-methyltransferases). In certain embodiments, the PF is PF05724.

In certain embodiments, the methyltransferase is characterized by an Ala, Ser or Thr at a position equivalent to position 122 of reference sequence of Uniprot entry Q145N6 (SEQ ID NO 1). In certain embodiments, the methyltransferase is characterized by a Thr, at a position equivalent to position 122 of reference sequence of Uniprot entry Q145N6 (SEQ ID NO 1).

In certain embodiments, the methyltransferase is characterized by the following amino acids at positions equivalent to reference sequence (SEQ ID NO 1) of Uniprot entry Q145N6:
- Position 23: Trp, Tyr or Phe, particularly Trp;
- Position 34: Trp, Tyr or Phe, particularly Trp;
- Position 59: Gly;
- Position 61: Gly;
- Position 80: Asp or Glu, particularly Asp;
- Position 122: Ala, Ser or Thr, particularly Thr;
- Position 126: Gly, Ala, Ser, Thr, particularly Ala;
- Position 163: Ala, Pro, Ser, particularly Pro.

In certain embodiments, the methyltransferase is derived from an organism selected from the group comprising *Ustilago maydis (uma), Aspergillus clavatus (acl), Dichomitus squalens (dsq), Vibri parhaemolyticus (vpa),* and *Kordia algicida (kal).* In certain embodiments, the methyltransferase is derived from the organism *uma.*

In certain embodiments, T-X and the methyltransferase are selected from any one of the following combinations:
- *vpa* and MMSa;
- *acl* and MeONs;
- *acl* and MeOTs;
- *acl* and dimethyl sulfite;
- *uma* and MeONs;
- *uma* and MeOTs;
- *uma* and dimethyl sulfite;
- *kal* and MeONs;
- *kal* and MeOTs;
- *dsq* and DMSa;
- *dsq* and dimethyl sulfite;

In certain embodiments, T-X and the methyltransferase are *uma* and MeOTs.

### Isotopes and salts

In certain embodiments, T comprises at least one isotope, particularly T is isotope-labelled methyl or -CH₂F.

In certain embodiments, T comprises at least one stable heavy isotope. In certain embodiments, T is stable isotope-labelled methyl or stable isotope-labelled -CH₂F. In certain embodiments, T is methyl comprising ¹³C and/or ²H. In certain embodiments, T is -CH₂¹⁹F.

In certain embodiments, T comprises at least one radioisotope. In certain embodiments, T is radioisotope-labelled methyl or radioisotope-labelled -CH₂F. In certain embodiments, T is methyl comprising ¹¹C, ¹⁴C and/or ³H. In certain embodiments, T is -CH₂¹⁸F.

Whenever charged moieties appear, it is understood that they are present with an adequate counterion. In certain embodiments, a negatively charged counterion is Cl⁻. In certain embodiments, a positively charged counterion is Na⁺.

### Further aspects of the invention

A second aspect of the invention relates to a method for alkylation of a substrate, the method comprising the steps:
a. providing a substrate and a dealkylated carrier compound CC;
b. in an alkylation step, alkylating CC via the method according to any one of the preceding claims by transferring the alkyl group T yielding an alkylated carrier compound T-CC;
c. in an alkyl-substrate production step, transferring an alkyl group from T-CC to the substrate catalyzed by an N-, C-, O-, S-, or P-specific methyltransferase yielding an alkylated substrate and a dealkylated carrier compound;
wherein at least a part of the dealkylated carrier compound CC of step c is recycled to step b to regenerate the alkylated carrier compound T-CC.

A further aspect of the invention relates to a kit comprising a carrier compound as described above, a methyltransferase as described above, and a sulfur-, or oxygen- or, selenium-based alkyl-group donor as described above, and a carrier compound as described above.

In certain embodiments, the kit additionally comprises an N-, C-, O-, S-, or P-specific methyltransferase.

In certain embodiments, the kit additionally comprises a substrate.

A further aspect of the invention relates to a use of a carrier compound as described above, a methyltransferase as described above, a sulfur-, or oxygen- or, selenium-based alkyl - group donor as described above, and an N-, C-, O-, S-, or P-specific methyltransferase for production of an alkylated substrate.

Wherever alternatives for single separable features such as, for example, a chemical moiety, an enzyme sequence or an isotope label are laid out herein as "embodiments", it is to be understood that such alternatives may be combined freely to form discrete embodiments of the invention disclosed herein. Thus, any of the alternative embodiments for a chemical moiety may be combined with any of the alternative embodiments of enzyme sequence and these combinations may be combined with any isotope label mentioned herein.

The invention further encompasses the following items.

### Items

1. A method for production of an alkylated carrier compound comprising the steps:
a. providing a dealkylated carrier compound CC;
b. in an alkylation step applying:
   i. an alkyl-group donor X; and
   ii. a methyltransferase transferring an alkyl group T from X to CC; yielding an alkylated carrier compound T-CC;
characterized in that the alkyl-group donor X is sulfur-, or selenium-, or oxygen-based.
2. The method according to item 1, wherein T is of the formula -CH₂-R⁰ or -CHF-R⁰ with R⁰ being selected from H, CN, F, Cl or an unsubstituted or substituted moiety MM being unfunctionalized or functionalized, wherein MM is selected from the group comprising alkyl, aryl, heteroaryl, alkyl-aryl, alkyl-heteroaryl;
wherein functionalized MM comprises an alkene, an alkyne, an ether, a thioether, an ester, disulfide, and/or a thioester moiety,
wherein substituted MM is substituted with one or two or three moieties selected from the group comprising halogen, OH, NH₂, N₃, CN, COOH, NO₂, CONH₂, SO₃⁻;
SO₂NH₂, epoxide;
particularly MM is selected from the group comprising alkyl, aryl, alkyl-aryl,
more particularly MM is alkyl.
3. The method according to item 2, wherein alkyl is C₁-C₁₄ alkyl, aryl is a monocyclic C₆ or bicyclic C₁₀ ring system, and heteroaryl is a monocyclic C₂-C₅ or bicyclic C₄-C₉ ring system
particularly wherein the aryl group is selected from the group comprising benzene, naphthalene;
particularly wherein the heteroaryl group is selected from the group comprising pyridine, pyrimidine, pyridazine, pyrazine, triazine, indole,
particularly alkyl is C₁-C₁₄ alkyl comprising 0, 1, 2, 3, 4, or 5 double bonds or comprising 0, 1, 2, or 3 triple bonds.
4. The method according to any one of the preceding items, wherein T-X is selected from the group comprising 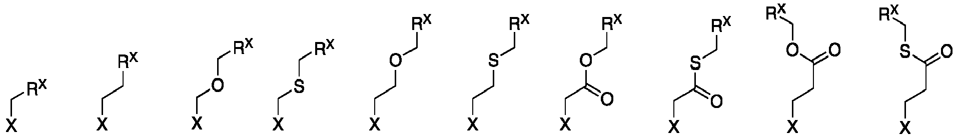 particularly T-X is selected from the group comprising 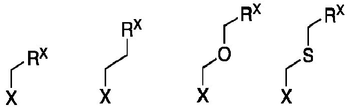 with R^{X} being selected from the group comprising H, F, OCH₃, OCH₂CH₃, CN, - COOH, NO₂, CF₃, CHF₂, CH₂F, CH₂Cl, CH₂Br, CONH₂, CH₂CO₂R^{X1},CH₃, (CH₂)ₘCH₃, CH(CH₃)₂, C₂H, CH₂C₂H, CH=CH₂, CH₂CH=CH₂, unsubstituted or R^{X2}-substituted O-aryl or aryl; with m being an integer selected from the group comprising 1 - 14; with R^{X1} being selected from the same group as R^{X} excluding CH₂CO₂R^{X1}; with each R^{X2} being independently selected from CH₃, (CH₂)ₘCH₃, CH(CH₃)₂, C(CH₃)₃, F, Cl, Br, I, OH, OR, NH₂, NHCH₃, NO₂, CN, CO₂, CF₃, CHF₂, CH₂F, CH₂Cl, CH₂Br, CONH₂, CH₂CO₂CH₃, SO₃⁻.
5. The method according to any one of the preceding items, wherein T-X is selected from the group comprising 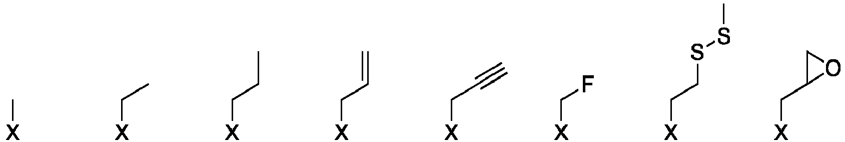 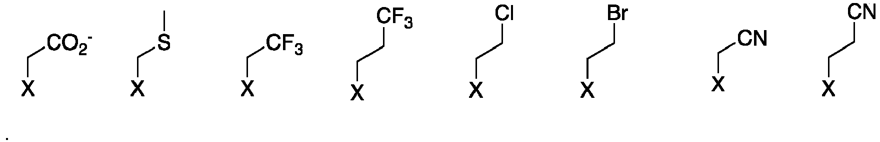 6. The method according to any one of the preceding items, wherein T is selected from the group comprising methyl, ethyl, propyl, propargyl, -CH₂F, particularly T is methyl.
7. The method according to any one of the preceding items, wherein CC is of the general formula: Z-P-Rib-Nuc,
wherein
   - Z is of the formula -CH₂-R^{Z} with R^{Z} being selected from H, or an unsubstituted or substituted moiety NN being unfunctionalized or functionalized, wherein NN is selected from the group comprising alkyl, aryl, heteroaryl, alkyl-aryl, alkyl-heteroaryl,
      wherein functionalized NN comprises an alkene, an alkyne, an ether, a thioether, an ester, and/or a thioester moiety,
      wherein substituted NN is substituted with one or two or three moieties selected from the group comprising halogen, CN, COOH, NO₂, NH₃, CONH₂, SO₃H;
      particularly NN is alkyl;
   - P is a sulfur atom or a selenium atom;
   - Rib is selected from the group comprising ribose, deoxyribose, 2-O-methyl ribose, carbaribose, 4-thioribose, particularly Rib is ribose;
   - Nuc is a nucleobase or a nucleobase derivative;
wherein P is connected to the 5' C atom of Rib, and Nuc is connected to the O atom of the 1' C atom of Rib.
8. The method according to any one of the preceding items, wherein CC is selected from the group comprising 5'-deoxy-5'-alkylthionucleoside and 5'-deoxy-5'-alkylselenonucleoside, with alkyl being selected from the group comprising methyl, fluormethyl, ethyl, propyl, 2-carboxy-2-aminoethyl, 3-aminopropyl, 3-hydroxypropyl, 3-carboxypropyl, 4-carboxybutyl, 4-aminobutyl, 4-hydroxybutyl, 3-carboxy-3-aminopropyl, 4-carboxy-4-aminobutyl,
particularly with alkyl being selected from the group comprising 2-carboxy-2-aminoethyl, 3-carboxy-3-aminopropyl.
9. The method according to any one of the preceding items, wherein CC is selected from the group comprising S-nucleosyl-homocysteine, Se-nucleosyl-homoselenocysteine, S-inosyl-L-homocysteine, Se-inosyl-L-homoselenocysteine, S-7-deazaadenosyl-L-homocysteine, S-7-deazaadenosyl-L-homoselenocysteine, S-formycinyl-L-homocysteine, Se-formycinyl-L-homoselenocysteine, S- aristeromycyl-L-homocysteine, Se- aristeromycyl-L-homoselenocysteine.
10. The method according to any one of the preceding items 1 to 8, wherein CC is selected from the group comprising

| Systematic Name |
|---|
| (*S*)-2-amino-4-((((2*S*,3*S*,4*R*,5*R*)-5-(6-amino-9*H*-purin-9-yl)-3,4-dihydroxytetrahydrofuran-2-yl)methyl)thio)-1-(λ¹-oxidaneyl)butan-1-one |
| (*R*)-4-((((2*S*,3*S*,4*R*,5*R*)-5-(6-amino-9*H*-purin-9-yl)-3,4-dihydroxytetrahydrofuran-2-yl)methyl)thio)-2-hyd roxybutanoate |
| (S)-4-((((2*S*,3*S*,4*R*,5*R*)-5-(4-amino-7*H*-pyrrolo[2,3-*d*]pyrimidin-7-yl)-3,4-dihydroxytetrahydrofuran-2-yl)methyl)thio)-2-ammoniobutanoate |
| (*S*)-2-ammonio-4-((((2*S*,3*S*,4*R*,5*R*)-3,4-dihydroxy-5-(6-hydroxy-9*H*-purin-9-yl)tetrahydrofuran-2-yl)methyl)thio)butanoate |
| (*S*)-2-ammonio-4-((((2*S*,3*S*,4*R*,5*R*)-3,4-dihydroxy-5-(9*H*-purin-9-yl)tetrahydrofuran-2-yl)methyl)thio)butanoate |
| (*S*)-4-((((2*S*,3*S*,4*R*,5*S*)-5-((*R*)-7-amino-3*H*-pyrazolo[4,3-*d*]pyrimidin-3-yl)-3,4-dihydroxytetrahydrofuran-2-yl)methyl)thio)-2-ammoniobutanoate |
| (S)-4-((((1S,2R,3S,4R)-4-(6-amino-9H-purin-9-yl)-2,3-dihydroxycyclopentyl)methyl)thio)-2-ammoniobutanoate |
| (*S*)-4-((((2S,3S,4R,5R)-5-(4-amino-1*H*-imidazo[4,5-c]pyridin-1-yl)-3,4-dihydroxytetrahydrofuran-2-yl)methyl)thio)-2-ammoniobutanoate |
| (*S*)-4-((((2S,3S,5R)-5-(6-amino-9*H*-purin-9-yl)-3-hydroxytetrahydrofuran-2-yl)methyl)thio)-2-ammoniobutanoate |
| (S)-4-((((2S,3S,4R,5R)-5-(4-amino-2-oxopyrimidin-1(2H)-yl)-3,4-dihydroxytetrahydrofuran-2-yl)methyl)thio)-2-ammoniobutanoate |
| (R)-3-((((2S,3S,4R,5R)-5-(6-amino-9H-purin-9-yl)-3,4-dihydroxytetrahydrofuran-2-yl)methyl)thio)-2-ammoniopropanoate |
| 3-((((2*S*,3*S*,4*R*,5*R*)-5-(6-amino-9*H*-purin-9-yl)-3,4-dihydroxytetrahydrofuran-2-yl)methyl)thio)propan-1-aminium |
| (2*R*,3*R*,4*S*,5*S*)-2-(6-amino-9*H*-purin-9-yl)-5-(((3-hydroxypropyl)thio)methyl)tetrahydrofuran-3,4-diol |
| 4-((((2S,3S,4R,5R)-5-(6-amino-9H-purin-9-yl)-3,4-dihydroxytetrahydrofuran-2-yl)methyl)thio)butanoate |
| (S)-4-((((2S,3S,4R,5R)-5-(6-amino-9*H*-purin-9-yl)-3-hydroxy-4-methoxytetrahydrofuran-2-yl)methyl)thio)-2-ammoniobutanoate |
| (2R,3R,4S,5S)-2-(6-amino-9H-purin-9-yl)-5-((methylthio)methyl)tetrahydrofuran-3,4-diol |
| (2R,3R,4S,5S)-2-(6-amino-9H-purin-9-yl)-5-((ethylthio)methyl)tetrahydrofuran-3,4-diol |
| (2R,3R,4S,5S)-2-(6-amino-9H-purin-9-yl)-5-((propylthio)methyl)tetrahydrofuran-3,4-diol |
| 5-((((2S,3S,4R,5R)-5-(6-amino-9H-purin-9-yl)-3,4-dihydroxytetrahydrofuran-2-yl)methyl)thio)pentanoate |
| (S)-4-((((2S,3*S*,4*R*,5*R*)-5-(6-amino-9*H*-purin-9-yl)-3,4-dihydroxytetrahydrofuran-2-yl)methyl)selanyl)-2-ammoniobutanoate |
| (*R*)-4-((((2*S*,3*S*,4*R*,5*R*)-5-(6-amino-9*H*-purin-9-yl)-3,4-dihydroxytetrahydrofuran-2-yl)methyl)selanyl)-2-hydroxybutanoate |
| (S)-4-((((1 S,2R,3S,4R)-4-(6-amino-9H-purin-9-yl)-2,3-dihydroxycyclopentyl)methyl)selanyl)-2-ammoniobutanoate |
| (S)-2-ammonio-4-((((2S,3S,4R,5R)-3,4-dihydroxy-5-(6-hydroxy-9H-purin-9-yl)tetrahydrofuran-2-yl)methyl)selanyl)butanoate |
| (S)-2-ammonio-4-((((2S,3S,4R,5R)-3,4-dihydroxy-5-(9H-purin-9-yl)tetrahydrofuran-2-yl)methyl)selanyl)butanoate |
| (S)-4-((((2S,3S,4R,5S)-5-((R)-7-amino-3H-pyrazolo[4,3-d]pyrimidin-3-yl)-3,4-dihydroxytetrahydrofuran-2-yl)methyl)selanyl)-2-ammoniobutanoate |
| (*S*)-4-((((1S,2R, 3S,4R)-4-(6-amino-9H-purin-9-yl)-2, 3-dihydroxycyclopentyl)methyl)selanyl)-2-ammoniobutanoate |
| (*S*)-4-((((2S,3S,4R,5R)-5-(4-amino-1H-imidazo[4,5-c]pyridin-1-yl)-3,4-dihydroxytetrahydrofuran-2-yl)methyl)selanyl)-2-ammoniobutanoate |
| (*S*)-4-((((2S,3S,5R)-5-(6-amino-9H-purin-9-yl)-3-hydroxytetrahydrofuran-2-yl)methyl)selanyl)-2-ammoniobutanoate |
| (*S*)-4-((((2S,3S,4R,5R)-5-(4-amino-2-oxopyrimidin-1(2H)-yl)-3,4-dihydroxytetrahydrofuran-2-yl)methyl)selanyl)-2-ammoniobutanoate |
| (*R*)-3-((((2*S*,3*S*,4*R*,5*R*)-5-(6-amino-9*H*-purin-9-yl)-3,4-dihydroxytetrahydrofuran-2-yl)methyl)selanyl)-2-ammoniopropanoate |
| 3-((((2*S*,3*S*,4*R*,5*R*)-5-(6-amino-9*H*-purin-9-yl)-3,4-dihydroxytetrahydrofuran-2-yl)methyl)selanyl)propan-1-aminium |
| (2*R*,3R,4S,5S)-2-(6-amino-9H-purin-9-yl)-5-(((3-hydroxypropyl)selanyl)methyl)tetrahydrofuran-3,4-diol |
| 4-((((2*S*,3*S*,4*R*,5*R*)-5-(6-amino-9*H*-purin-9-yl)-3,4-dihydroxytetrahydrofuran-2-yl)methyl)selanyl)butanoate |
| (*S*)-4-((((2*S*,3*S*,4*R*,5*R*)-5-(6-amino-9*H*-purin-9-yl)-3-hydroxy-4-methoxytetrahydrofuran-2-yl)methyl)selanyl)-2-ammoniobutanoate |
| (2*R*,3*R*,4*S*,5*S*)-2-(6-amino-9*H*-purin-9-yl)-5-((methylselanyl)methyl)tetrahydrofuran-3,4-diol |
| (2*R*,3*R*,4*S*,5*S*)-2-(6-amino-9*H*-purin-9-yl)-5-((ethylselanyl)methyl)tetrahydrofuran-3,4-diol |
| (2*R*,3*R*,4*S*,5*S*)-2-(6-amino-9*H*-purin-9-yl)-5-((propylselanyl)methyl)tetrahydrofuran-3,4-diol |
| 5-((((2*S*,3*S*,4*R*,5*R*)-5-(6-amino-9*H*-purin-9-yl)-3,4-dihydroxytetrahydrofuran-2-yl)methyl)selanyl)pentanoate |

particularly CC is selected from the group comprising:

| Systematic Name |
|---|
| (*S*)-2-amino-4-((((2*S*,3*S*,4*R*,5*R*)-5-(6-amino-9*H*-purin-9-yl)-3,4-dihydroxytetrahydrofuran-2-yl)methyl)thio)-1-(λ¹-oxidaneyl)butan-1-one |
| (*R*)-4-((((2*S*,3*S*,4*R*,5*R*)-5-(6-amino-9*H*-purin-9-yl)-3,4-dihydroxytetrahydrofuran-2-yl)methyl)thio)-2-hydroxybutanoate |
| (*S*)-4-((((2*S*,3*S*,4*R*,5*R*)-5-(4-amino-7*H*-pyrrolo[2,3-*d*]pyrimidin-7-yl)-3,4-dihydroxytetrahydrofuran-2-yl)methyl)thio)-2-ammoniobutanoate |
| (S)-2-ammonio-4-((((2S,3S,4R,5R)-3,4-dihydroxy-5-(6-hydroxy-9H-purin-9-yl)tetrahydrofuran-2-yl)methyl)th io)butanoate |
| (*S*)-2-ammonio-4-((((2*S*,3*S*,4*R*,5*R*)-3,4-dihydroxy-5-(9*H*-purin-9-yl)tetrahydrofuran-2-yl)methyl)thio)butanoate |
| (S)-4-((((2S,3S,4R,5S)-5-((R)-7-amino-3H-pyrazolo[4,3-d]pyrimidin-3-yl)-3,4-dihydroxytetrahydrofuran-2-yl)methyl)thio)-2-ammoniobutanoate |
| (S)-4-((((1S,2R,3S,4R)-4-(6-amino-9H-purin-9-yl)-2,3-dihydroxycyclopentyl)methyl)thio)-2-ammoniobutanoate |
| (*S*)-4-((((2*S,*3*S*,4*R*,5*R*)-5-(4-amino-1*H*-imidazo[4,5-*c*]pyridin-1-yl)-3,4-dihydroxytetrahydrofuran-2-yl)methyl)thio)-2-ammoniobutanoate |
| (*S*)-4-((((2*S*,3*S*,5*R*)-5-(6-amino-9*H*-purin-9-yl)-3-hydroxytetrahydrofuran-2-yl)methyl)thio)-2-ammoniobutanoate |
| (*S*)-4-((((2*S*,3*S*,4*R*,5*R*)-5-(4-amino-2-oxopyrimidin-1(2*H*)-yl)-3,4-dihydroxytetrahydrofuran-2-yl)methyl)thio)-2-ammoniobutanoate |
| (*R*)-3-((((2*S*,3*S*,4*R*,5*R*)-5-(6-amino-9*H*-purin-9-yl)-3,4-dihydroxytetrahydrofuran-2-yl)methyl)thio)-2-ammoniopropanoate |
| 3-((((2S,3S,4R,5R)-5-(6-amino-9H-purin-9-yl)-3,4-dihydroxytetrahydrofuran-2-yl)methyl)thio)propan-1-aminium |
| (2*R*,3*R*,4*S*,5*S*)-2-(6-amino-9*H*-purin-9-yl)-5-(((3-hydroxypropyl)thio)methyl)tetrahydrofuran-3,4-diol |
| 4-((((2S,3S,4R,5R)-5-(6-amino-9H-purin-9-yl)-3,4-dihydroxytetrahydrofuran-2-yl)methyl)thio)butanoate |
| (S)-4-((((2S,3S,4R,5R)-5-(6-amino-9H-purin-9-yl)-3-hydroxy-4-methoxytetrahydrofuran-2-yl)methyl)thio)-2-ammoniobutanoate |
| (2*R*,3*R*,4*S*,5*S*)-2-(6-amino-9*H*-purin-9-yl)-5-((methylthio)methyl)tetrahydrofuran-3,4-diol |
| (2*R*,3*R*,4*S*,5*S*)-2-(6-amino-9*H*-purin-9-yl)-5-((ethylthio)methyl)tetrahydrofuran-3,4-diol |
| (2*R*,3*R*,4*S*,5*S*)-2-(6-amino-9*H*-purin-9-yl)-5-((propylthio)methyl)tetrahydrofuran-3,4-diol |
| 5-((((2S,3S,4R,5R)-5-(6-amino-9H-purin-9-yl)-3,4-dihydroxytetrahydrofuran-2-yl)methyl)thio)pentanoate |

11. The method according to any one of the preceding items, wherein CC is S-adenosyl-L-homocysteine (SAH).
12. The method according to any one of the preceding items, wherein X comprises a moiety selected from the group comprising sulfate, sulfonate, sulfite, sulfinate,
thiosulfate, thiosulfonate, thiosulfinate, selenite, selenate, selenonate, seleninate, wherein T is attached to said moiety,
particularly wherein X comprises a moiety selected from the group comprising sulfate, sulfonate, sulfite, sulfinate, thiosulfate, thiosulfonate, thiosulfinate.
13. The method according to any one of the preceding items, wherein X is selected from the group comprising alkylsulfate, arylsulfate, heteroarylsulfate, alkylsulfonate, arylsulfonate, heteroarylsulfonate, alkylsulfite, arylsulfite, heteroarylsulfite, alkylsulfinate, arylsulfinate, heteroarylsulfinate, alkylthiosulfate, arylthiosulfate, heteroarylthiosulfate, alkylthiosulfonate, arylthiosulfonate, heteroarylthiosulfonate, alkylthiosulfinate, arylthiosulfinate, heteroarylthiosulfinate.
14. The method according to item 13, wherein the aryl group is a substituted or unsubstituted monocyclic or bicyclic compound;
particularly wherein the aryl group is selected from the group comprising benzene, naphthalene.
15. The method according to item 13, wherein the heteroaryl group is a substituted or unsubstituted monocyclic or bicyclic compound;
particularly wherein the heteroaryl group is selected from the group comprising pyridine, pyrimidine, pyridazine, pyrazine, triazine, indole.
16. The method according to item 13, wherein the alkyl group is a substituted or unsubstituted linear or cyclic C₁-C₁₅ hydrocarbon.
17. The method according to any one of the preceding items, wherein T-X is selected from the group comprising 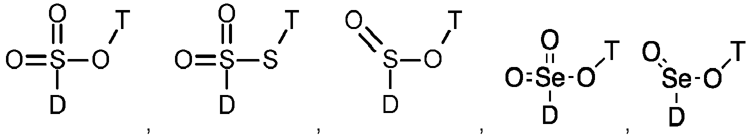 with D being selected from -R^{D1}, -OR^{D2}, -SR^{D2}, -NR^{D2}R^{D3}, with
- R^{D1} being selected from the group comprising an unsubstituted or substituted moiety LL being unfunctionalized or functionalized, wherein LL is selected from the group comprising alkyl, aryl, heteroaryl, alkyl-aryl, alkyl-heteroaryl, wherein functionalized LL comprises an alkene, an alkyne, an ether, a thioether, an ester, and/or a thioester moiety,
   wherein substituted LL is substituted with one or two or three moieties selected from the group comprising halogen, CN, COOH, NO₂, NH₃, CONH₂, SO₃H;
- R^{D2} and R^{D3} being independently selected from the group comprising H or R^{D1}.
18. The method according to any one of the preceding items,
wherein T-X is selected from the group comprising
   a. 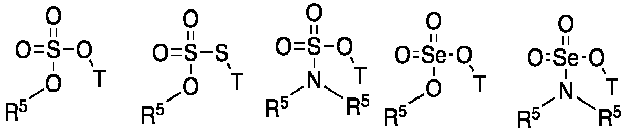 with each R^{s} being independently selected from the group comprising H, CH₃, (CH₂)ₕCH₃, CH(CH₃)₂, C(CH₃)₃, CN, CF₃, CHF₂, CH₂F, CH₂Cl, CH₂Br, CH₂NO₂, CH₂CO₂CH₃, C₆H₁₁;
   b. 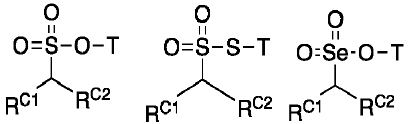 with R^{C1} and R^{C2} being independently selected from the group comprising H, CH₃, (CH₂)ₕCH₃, CH(CH ₃)₂, C(CH₃)₃, F, Cl, Br, I, OH, OR⁶, NH₂, NHR⁶, NO₂, CN, CO₂-, CF₃, CHF₂, CH₂F, CH₂Cl, CH₂Br, CONH₂, CH₂CO₂CH₃₁ C₆H₁₁, SO₃⁻
   c. 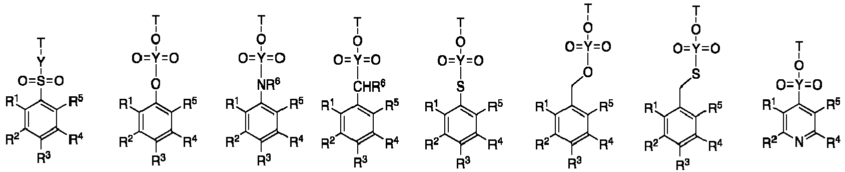 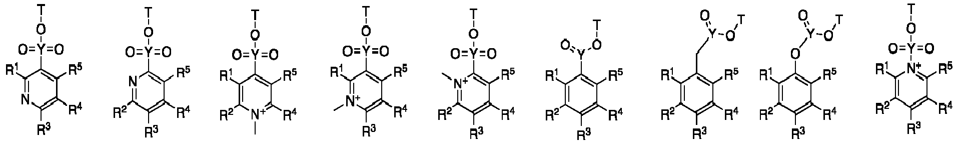
      With Y = S or Se; with R¹ - R⁵ being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂, CF₃, CHF₂, CH₂F, CH₂Cl, CH₂Br, CONH₂, CH₂CO₂CH₃, S(CH₃)₂⁺Cl, N(CH₃)₃⁺Cl, SO₃Na
      in particular from H, CH₃, F, Cl, Br, OMe, CF₃, NO₂, CN, CO₂Me, CH(CH₃)₂, S(CH₃)₂⁺Cl, N(CH₃)₃⁺Cl, C(CH₃)₃, more particularly from H, CH₃, F, Cl, Br, OMe, CF₃, NO₂, CN,
particularly wherein T-X is selected from the group comprising
   a. 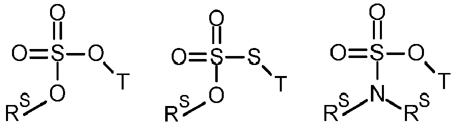 with each R^{S} being independently selected from the group comprising H, CH₃, (CH₂)ₕCH₃, CH(CH₃)₂, C(CH₃)₃, CN, CF₃, CHF₂, CH₂F, CH₂Cl, CH₂Br, CH₂NO₂, CH₂CO₂CH₃, C₆H₁₁;
   b. 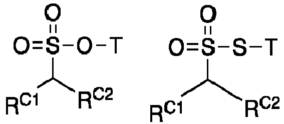 with R^{C1} and R^{C2} being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂-, CF₃, CHF₂, CH₂F, CH₂Cl, CH₂Br, CONH₂, CH₂CO₂CH₃, C₆H₁₁, SO₃⁻
   c. 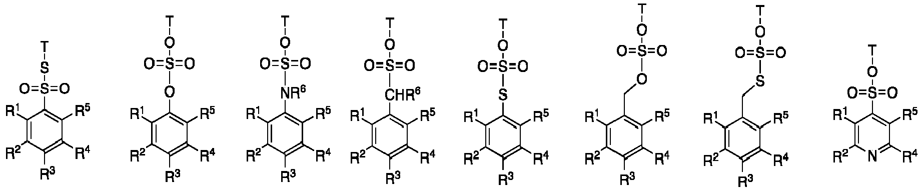 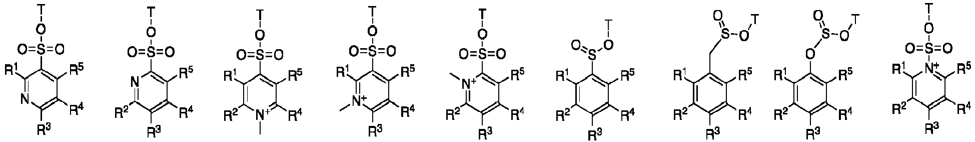
      with R¹ - R⁵ being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂, CF₃, CHF₂, CH₂F, CH₂Cl, CH₂Br, CONH₂, CH₂CO₂CH₃, S(CH₃)₂⁺, N(CH₃)₃⁺, SO₃⁻;
      in particular from H, CH₃, F, Cl, Br, OMe, CF₃, NO₂, CN, CO₂Me, CH(CH₃)₂, S(CH₃)₂⁺, N(CH₃)₃⁺, C(CH₃)₃, more particularly from H, CH₃, F, Cl, Br, OMe, CF₃, NO₂, CN,
with each R⁶ being independently selected from the group comprising H, CH_{3;}
with h being an integer selected from the group comprising 1 - 14.
19. The method according to any one of the preceding items, wherein T-X is selected from the group comprising
a. 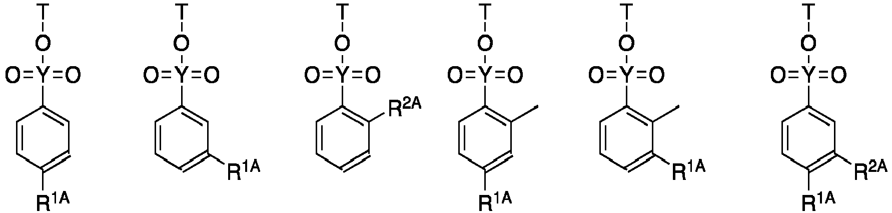 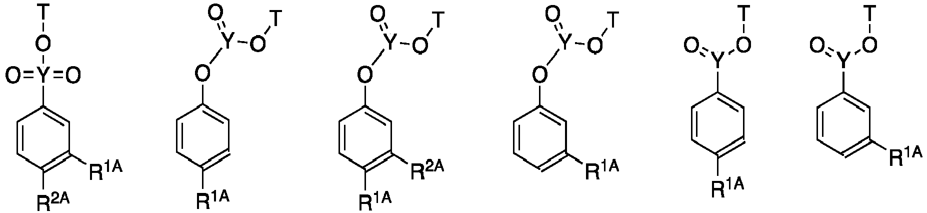
   With Y = S or Se; with R^{1A} being selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃,
   with R^{2A} being selected from the group comprising H, CH₃, F, Cl, Br, OMe, CF₃, NO₂, CN,
b. 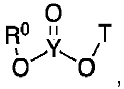 particularly: 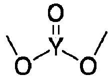 With Y = S or Se; with R^{O} being selected from the group comprising H, CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃, C₆H₁₁;
c. 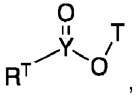 particularly 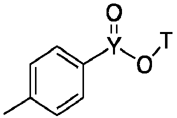 With Y = S or Se; with R^{T} being selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁
particularly wherein T-X is selected from the group comprising
a. 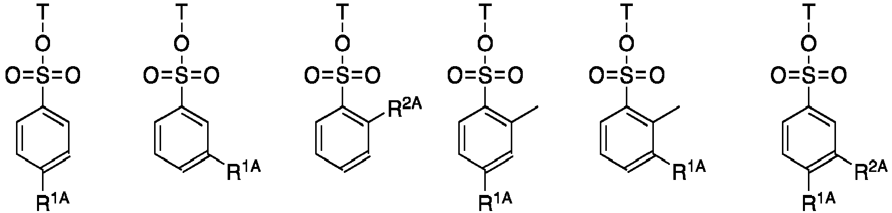 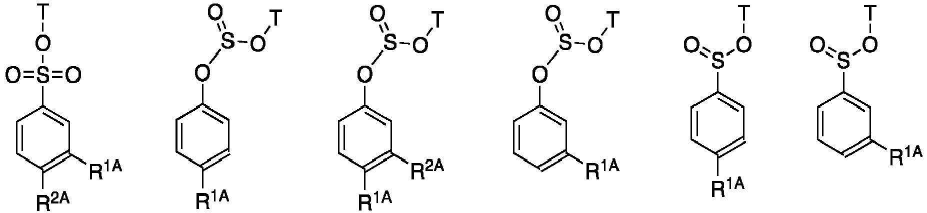
   with R^{1A} being selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃,
   with R^{2A} being selected from the group comprising H, CH₃, F, Cl, Br, OMe, CF₃, NO₂, CN,
b. 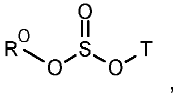 particularly: 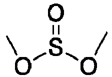 with R^{O} being selected from the group comprising H, CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃, C₆H₁₁;
c. 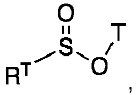 particularly 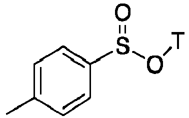
   with R^{T} being selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁
   with k being an integer selected from the group comprising 1 - 14.
20. The method according to any one of the preceding items 1 to 16, wherein T-X is selected from the group comprising a cyclic sulfate, a cyclic sulfite, a sultone, a cyclic sulfamidate, a thiirane-1-oxide, a lactone or a thiolactone.
21. The method according to item 20, wherein T-X is selected from the group comprising 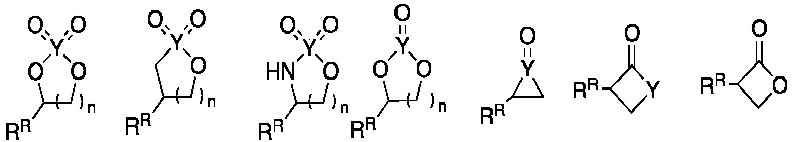
with Y = S or Se;
particularly T-X is selected from the group comprising 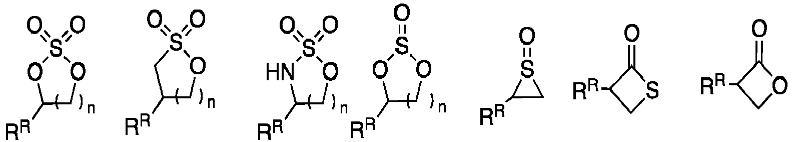
more particularly T-X is selected from the group comprising 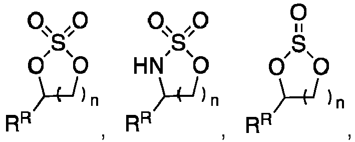 most particularly T-X is 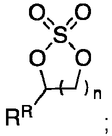
with R^{R} being selected from the group comprising H, OR, NO₂, CN, COOH, CF₃, CHF₂, CH₂F, CH₂Cl, CH₂Br, CONH₂, CH₂CO₂Me, CH₃, (CH₂)ₙCH₃, with n being an integer selected from the group comprising 1 - 14.
22. The method according to any one of the preceding items, wherein X is sulfur-based.
23. The method according to any one of the preceding items, wherein X comprises a sulfonate or a sulfate moiety.
24. The method according to any one of the preceding items, wherein T-X is selected from the group comprising methyl toluenesulfonate (MeOTs), methyl 4-nitrobenzenesulfonate (MeONs), dimethyl sulfate (DMSa), or methyl methanesulfonate (MMSa), dimethyl sulfite, S-methyl thiosulfate, particularly the alkyl-group donor is MeOTs.
25. The method according to any one of the preceding items, wherein the methyltransferase is an S-adenosylmethionine (SAM)-dependent methyltransferase.
26. The method according to item 25, wherein the SAM-dependent methyltransferase is capable of methylating anionic nucleophiles, particularly anionic nucleophiles selected from the group comprising halides, phosphates, sulfates, sulfonates, sulfites, thiolates, sulfinic acids, carboxylates, N-oxides, cyanate, thiocyanate.
27. The method according to any one of the preceding items, wherein the methyltransferase belongs to a protein family (PF) or is derived from a protein belonging to a PF, wherein the PF is selected from the group comprising PF05724 (including thiopurine methyltransferases, halide methyltransferases and N-oxide methyltransferases), PF03492 (including carboxyl methyltransferases), PF08241 (including thiol S-methyltransferases).
28. The method according to item 27, wherein the PF is PF05724.
29. The method according to item 28, wherein the methyltransferase is characterized by an Ala, Ser or Thr, particularly Thr, at a position equivalent to position 122 of reference sequence of Uniprot entry Q145N6,
particularly the methyltransferase is characterized by the following amino acids at positions equivalent to reference sequence (SEQ ID NO 1) of Uniprot entry Q145N6:
- Position 23: Trp, Tyr or Phe, particularly Trp;
- Position 34: Trp, Tyr or Phe, particularly Trp;
- Position 59: Gly;
- Position 61: Gly;
- Position 80: Asp or Glu, particularly Asp;
- Position 122: Ala, Ser or Thr, particularly Thr;
- Position 126: Gly, Ala, Ser, Thr, particularly Ala;
- Position 163: Ala, Pro, Ser, particularly Pro.
30. The method according to item 28 or 29, wherein the methyltransferase is derived from an organism selected from the group comprising *Ustilago maydis (uma), Aspergillus clavatus (acl), Dichomitus squalens (dsq), Vibri parhaemolyticus (vpa),* and *Kordia algicida (kal),* particularly from the organism *uma.*
31. The method according to any one of the preceding items, wherein T comprises at least one isotope, particularly T is isotope-labelled methyl or -CH₂F.
32. The method according to any one of the preceding items, wherein T comprises at least one stable heavy isotope, particularly T is stable heavy isotope-labelled methyl or stable heavy isotope-labelled -CH₂F, more particularly T is methyl comprising ¹³C and/or ²H or T is -CH₂¹⁹F.
33. The method according to any one of the preceding items, wherein T comprises at least one radioisotope, particularly T is radioisotope-labelled methyl or radioisotope-labelled -CH₂F, more particularly T is methyl comprising ¹¹C, ¹⁴C and/or ³H or T is -CH₂¹⁸F.
34. A method for alkylation of a substrate, the method comprising the steps:
a. providing a substrate and a dealkylated carrier compound CC;
b. in an alkylation step, alkylating CC via the method according to any one of the preceding items yielding an alkylated carrier compound T-CC;
c. in an alkyl-substrate production step, transferring an alkyl group from T-CC to the substrate catalyzed by an N-, C-, O-, S-, or P-specific methyltransferase yielding an alkylated substrate and a dealkylated carrier compound;
wherein at least a part of the dealkylated carrier compound CC of step c is recycled to step b to regenerate the alkylated carrier compound T-CC.
35. A kit comprising a carrier compound as described in items 1 and 2, a methyltransferase as described in items 1, and 25-30, and a sulfur-, or oxygen- or, selenium-based alkyl-group donor as described in items 1, and 12-24, and a carrier compound as described in items 1, and 7-11.
36. The kit according to item 35 additionally comprising an N-, C-, O-, S-, or P-specific methyltransferase.
37. The kit according to item 35 or 36 additionally comprising a substrate.
38. Use of a carrier compound as described in items 1, and 7-11, a methyltransferase as described in items 1, and 25-30, a sulfur-, or oxygen- or, selenium-based alkyl -group donor as described in items 1, and 12-24, and an N-, C-, O-, S-, or P-specific methyltransferase for production of an alkylated substrate.

The invention is further illustrated by the following examples and figures, from which further embodiments and advantages can be drawn. These examples are meant to illustrate the invention but not to limit its scope.

### Description of the Figures

- Fig. 1: shows Methyltransferase (MT)-catalyzed methylation of nucleophilic substrates. Halide methyltransferase (HMT)-catalyzed methylation of S-adenosyl homocysteine (SAH) with methyl iodide (Mel) as methyl donor provides a method for *in situ* regeneration of SAM. This report explores the possibility to replace Mel with non-volatile methyldonors such as dimethyl sulfate (DMSa), methyl methanosulfonate (MMSa), methyl toluene sulfonate (MeOTs) methyl 4-nitrobenzenesulfonate (MeONs).
- Fig. 2: shows **left:** Overall structure of *Bx*HMT in complex with SAH (gray) and chloride (green). **Middle:** Cross section of the active site cavity of *Bx*HMT shows that the halide binding site is connected to bulk solvent through an open tunnel. **Right:** In *At*HMT the N-terminal alpha-helix (red) closes off the halide binding site.

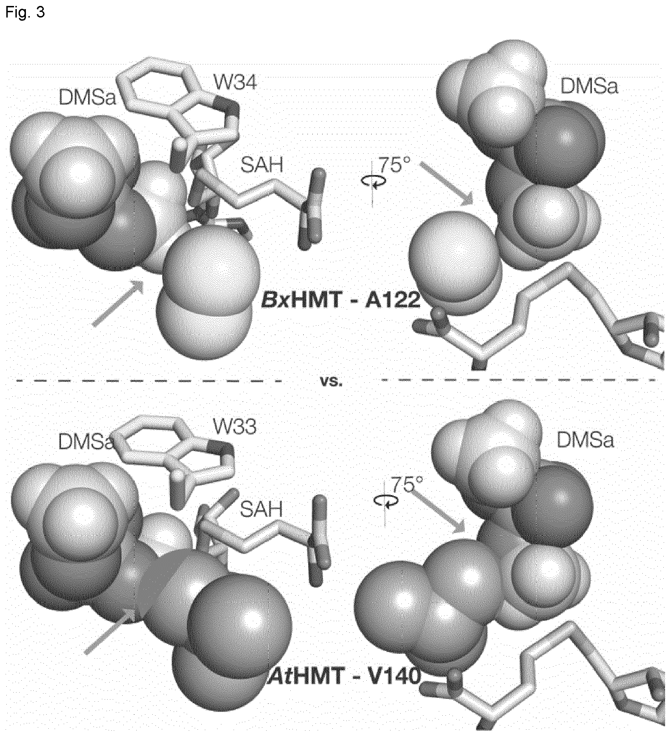

- Fig. 3: shows side by side comparison of *Bx*HMT **(top)** and *At*HMT **(bottom)** DMSa model structure with focus on the Ala122 or Val140, respectively. Ala122 or Val140 and DMSa in space filling representation indicate, that DMSa can occupy the position next to Ala122 to top of the sulfonium without steric repulsion, whereas DMSa clashes into Val140 in *At*HMT (arrow).

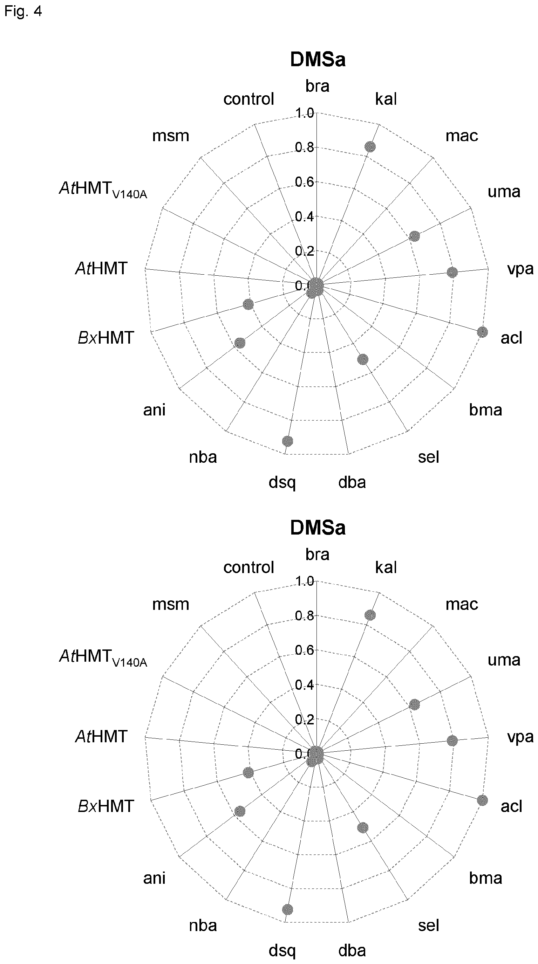

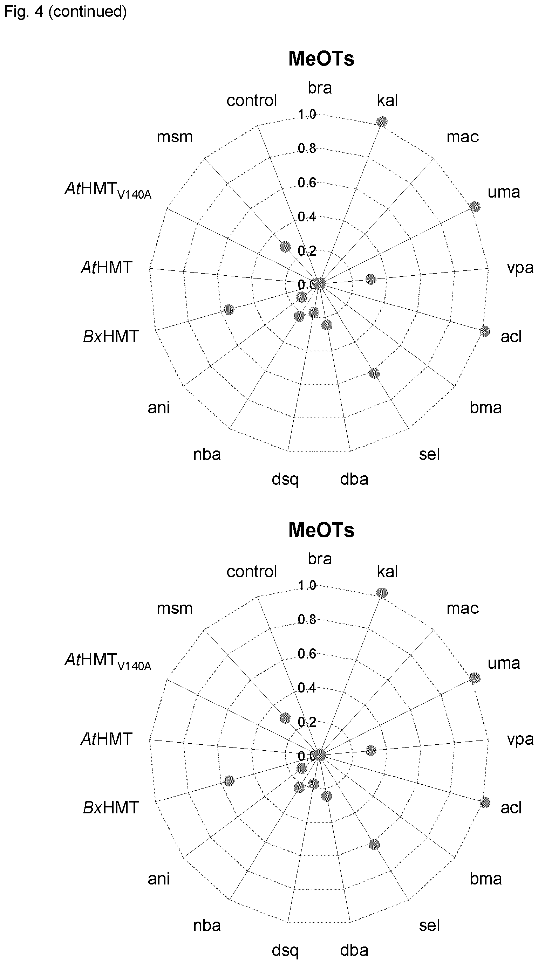

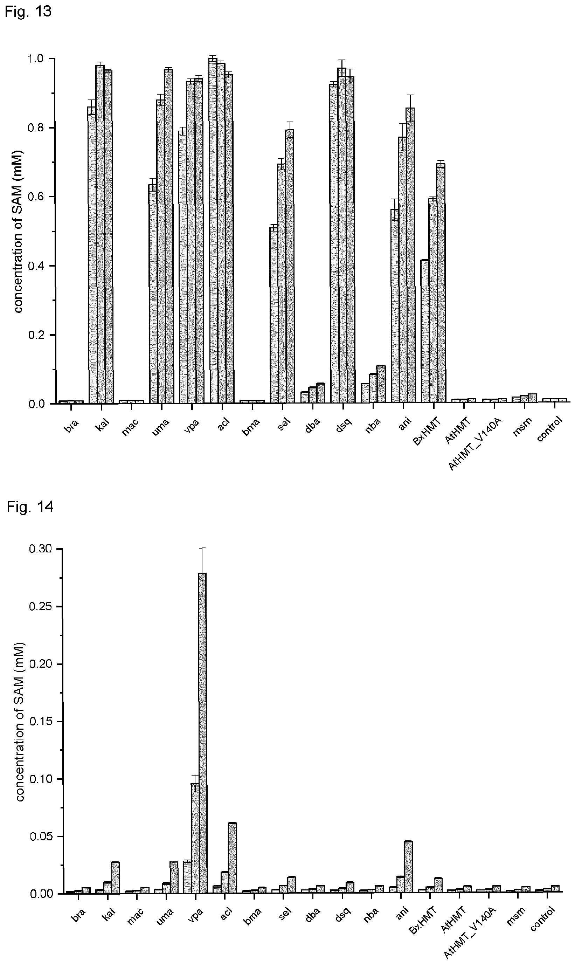

- Fig. 4: shows radar charts displaying the ability of TPMTs (10 µM) to produce SAM (radial axis: [SAM] in mM) in reactions containing 1 mM SAH and 2 mM DMSa, MMSa MeOTs or MeONs in 50 mM phosphate buffer pH 8.0 at 25°C. SAM concentrations were determined after 0.5 h (DMSa, and MeONs), 1 h (MeOTs) or 24 h (MMSa). The given values are averages of three independent measurements. The associated error is less than 30 % of the average value. Quantitative data is show in Figures 13- 15.

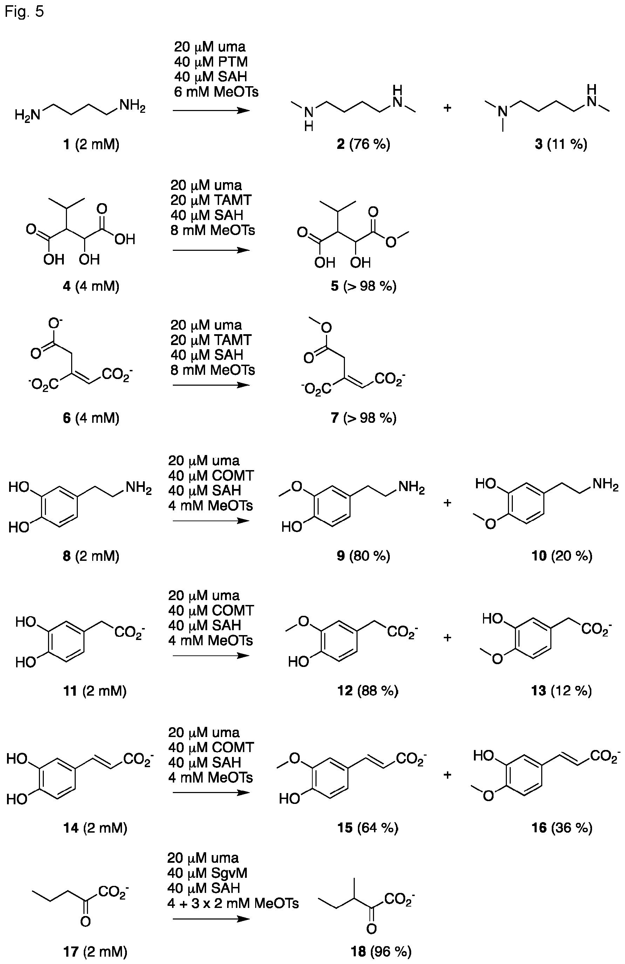

- Fig. 5: shows Enzyme-catalyzed methylation using uma-catalyzed regeneration of SAM. Compounds were identified by HR-ESI-MS and yields were determined by ¹H NMR.

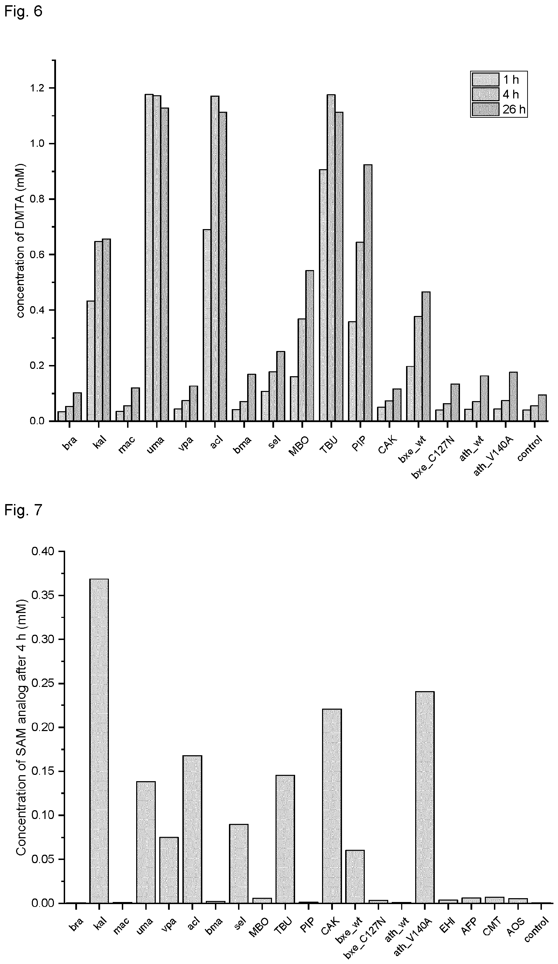

- Fig. 6: shows bar graph of HMT-catalyzed 5'-MTA methylation using MeOTs as a methyl group donor. 200 µL reactions containing 50 mM sodium phosphate buffer (pH 8.0), 1 mM 5'-MTA, 2 mM MeOTs and 10 µM different HMT were incubated at 25 °C. The reactions were quenched by 1% TFA in water and the concentrations of the product DMTA were monitored by HPLC at 1 hour (orange), 4 hours (green) and 26 hours (purple).
- Fig. 7: shows HPLC analysis monitoring TPMT-catalyzed SAH alkylation using ethylene sulfate as an alkyl group donor.

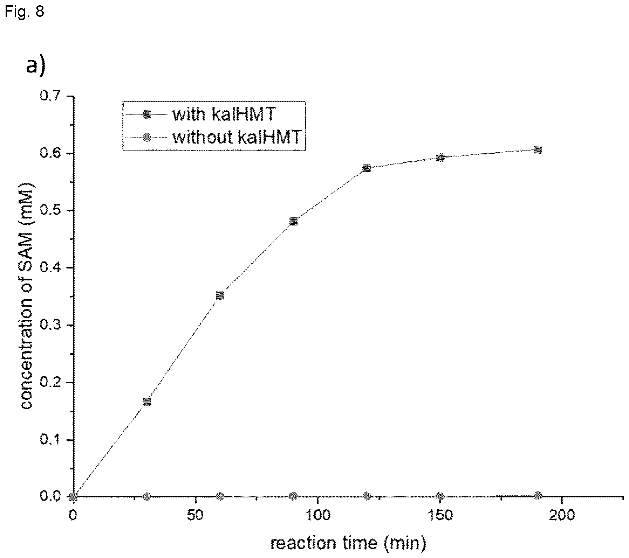

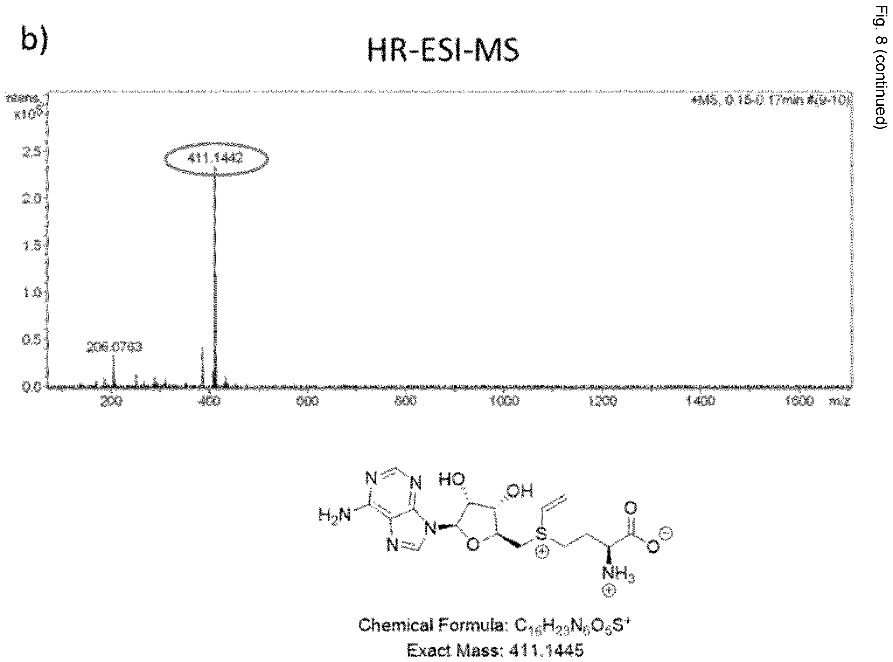

- Fig. 8: shows kaITPMT-catalyzed SAH alkylation using ethylene sulfate as an alkyl group donor. a) HPLC analysis; b) HR-ESI-MS spectrum of the product and its possible structure.

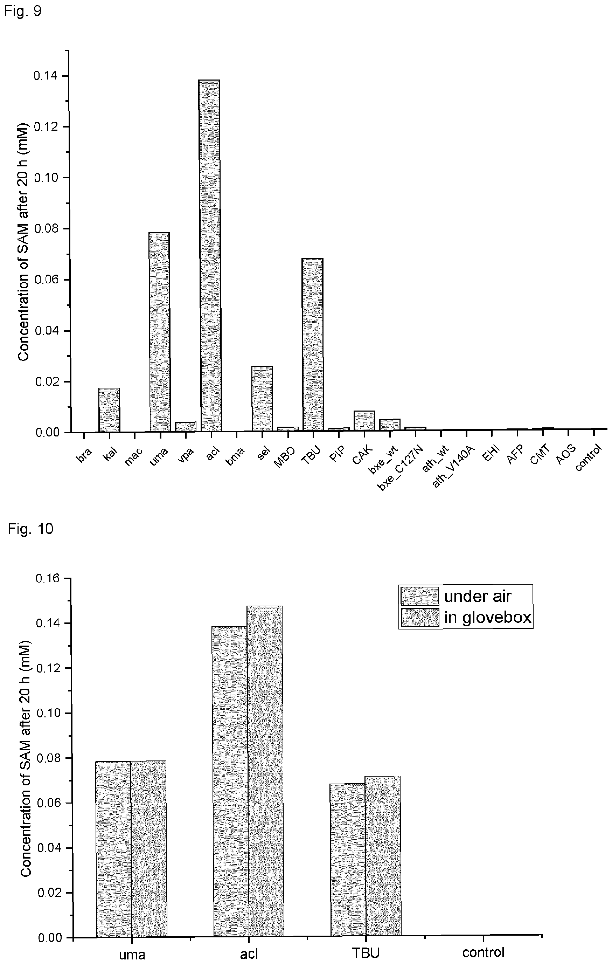

- Fig. 9: shows HPLC analysis monitoring TPMT-catalyzed SAH methylation using dimethyl sulfite as a methyl group donor.
- Fig. 10: shows HPLC analysis monitoring TPMT-catalyzed SAH methylation using dimethyl sulfite as a methyl group donor in different conditions.

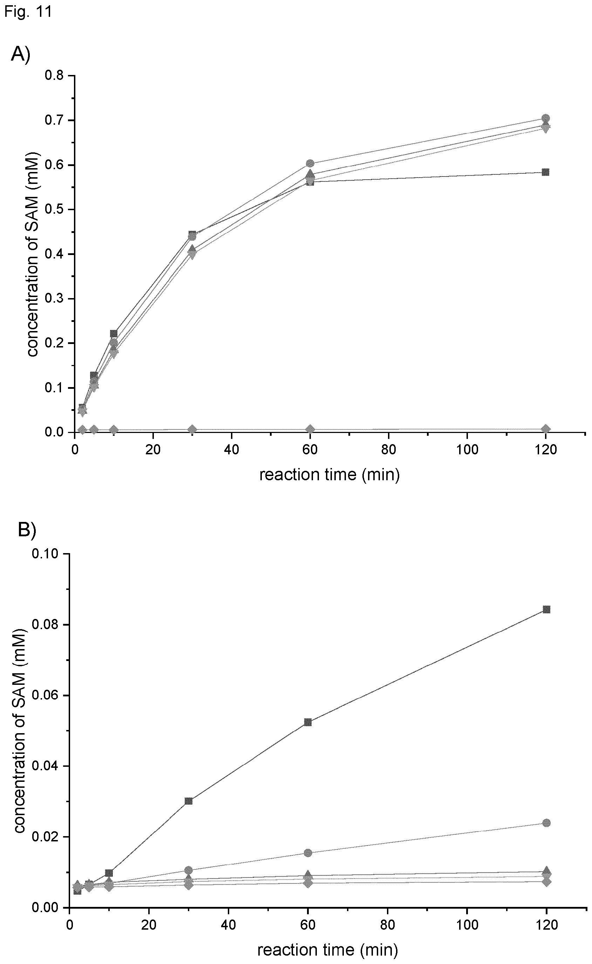

- Fig. 11: shows time-dependent methylation of SAH (1 mM) by different HMT using dimethyl sulfate (DMSa, 2 mM) as a methyl group donor in the absence or presence of sodium iodide (black: with 10 mM Nal, red: with 1.0 mM Nal, blue: with 0.1 mM Nal, green: no Nal, purple: no enzyme). In addition, the reactions contained 50 mM sodium phosphate buffer (pH 8.0) and were incubated at 25 °C. The concentration of SAM was determined by HPLC. A) with 10 µM *Bx*HMT; B) with 10 µM *At*HMT.

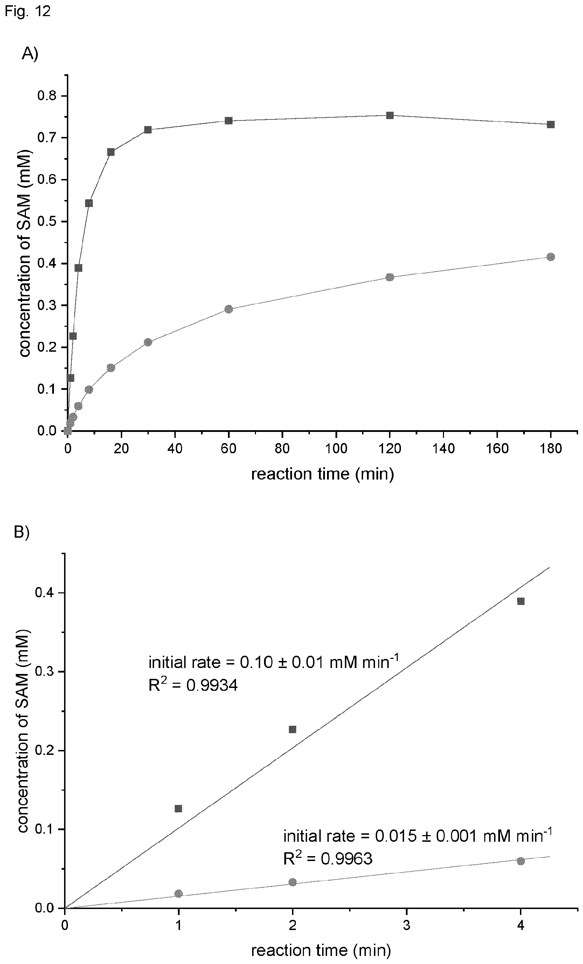

- Fig. 12: shows A) time-dependent methylation of SAH (1 mM) by *At*HMT (1 µM, black) or *At*HMT_{V140A} (1 µM, red) using methyl iodide (2 mM, Mel) as a methyl group donor in 50 mM sodium phosphate buffer (pH 8.0) at 25 °C. The concentration of SAM was determined by HPLC; B) Initial rates of these reactions were estimated from the slopes of linear-fit curves of the points at 1, 2 and 4 minutes.
- Fig. 13: shows bar graph of TPMT-catalyzed SAH methylation using DMSa as a methyl group donor. 200 µL reactions containing 50 mM sodium phosphate buffer (pH 8.0), 1 mM SAH, 2 mM DMSa and 10 µM different TPMT were incubated at 25 °C. The reactions were quenched by 1% TFA in water and the concentrations of the product SAM were monitored by HPLC at 0.5 hour (orange), 1 hour (green) and 2 hours (purple). The given values are averages of three independent measurements and error bars represent the standard deviations.
- Fig. 14: shows bar graph of TPMT-catalyzed SAH methylation using MMSa as a methyl group donor. 200 µL reactions containing 50 mM sodium phosphate buffer (pH 8.0), 1 mM SAH, 2 mM MMSa and 10 µM different TPMT were incubated at 25 °C. The reactions were quenched by 1% TFA in water and the concentrations of the product SAM were monitored by HPLC at 1 hour (orange), 4 hours (green) and 24 hours (purple). The given values are averages of three independent measurements and error bars represent the standard deviations.

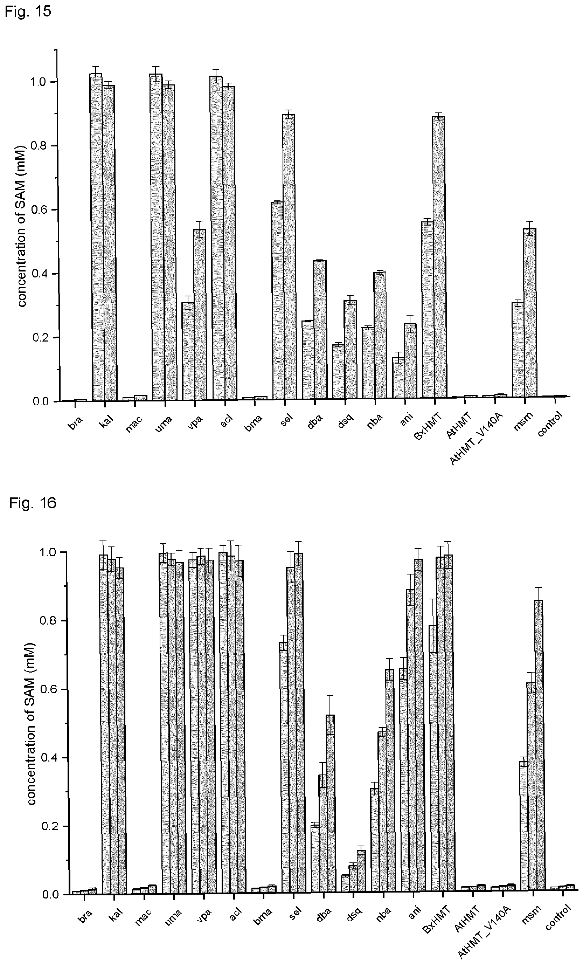

- Fig. 15: shows bar graph of TPMT-catalyzed SAH methylation using MeOTs as a methyl group donor. 200 µL reactions containing 50 mM sodium phosphate buffer (pH 8.0), 1 mM SAH, 2 mM MeOTs and 10 µM different TPMT were incubated at 25 °C. The reactions were quenched by 1% TFA in water and the concentrations of the product SAM were monitored by HPLC at 1 hour (orange) and 2 hours (green). The given values are averages of three independent measurements and error bars represent the standard deviations.
- Fig. 16: shows bar graph of TPMT-catalyzed SAH methylation using MeONs as a methyl group donor. 200 µL reactions containing 50 mM sodium phosphate buffer (pH 8.0), 1 mM SAH, 2 mM MeONs and 10 µM different TPMT were incubated at 25 °C. The reactions were quenched by 1% TFA in water and the concentrations of the product SAM were monitored by HPLC at 0.5 hour (orange), 1 hour (green) and 2 hours (purple). The given values are averages of three independent measurements and error bars represent the standard deviations.

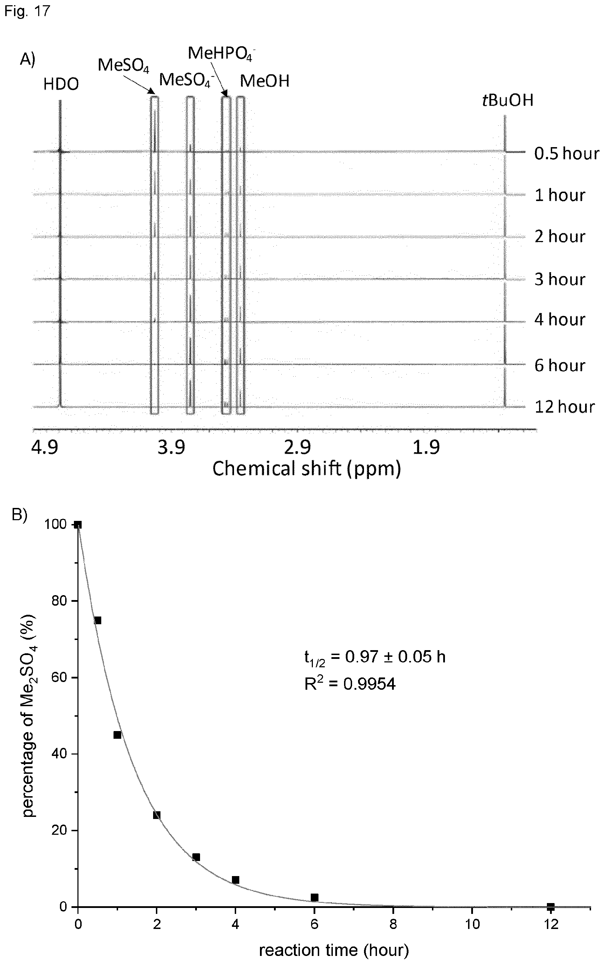

- Fig. 17: shows stability test of DMSa in phosphate buffer. A) Stack of ¹H NMR spectra of 4 mM DMSa in 50 mM sodium phosphate buffer in D₂O (pD 8.4) after 0.5 to 12 hours; B) The graph of percentage of DMSa in all methyl species over time. The calculated half-life of DMSa at pD 8.4 was (0.97 ± 0.05) hour.

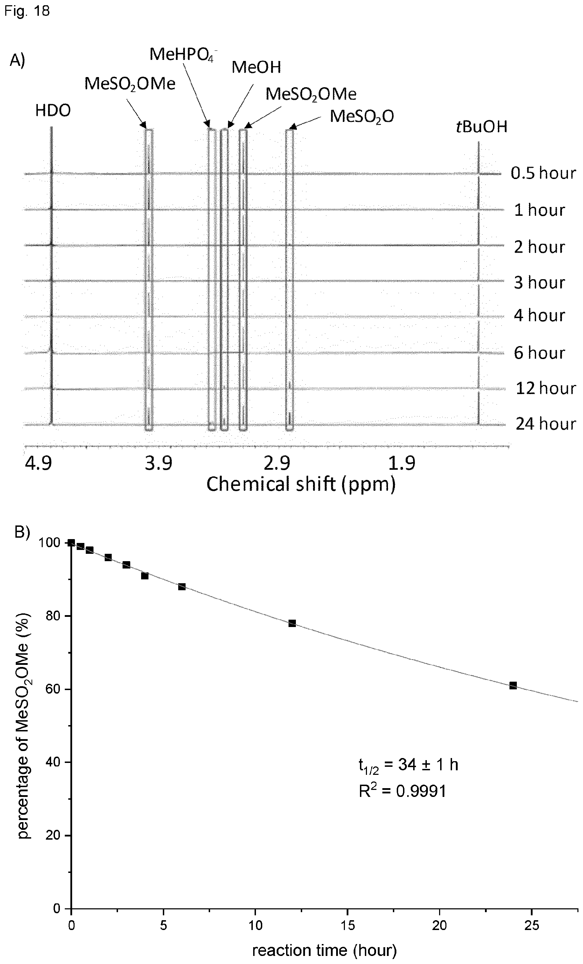

- Fig. 18: shows stability test of MMSa in phosphate buffer. A) Stack of ¹H NMR spectra of 4 mM MMSa in 50 mM sodium phosphate buffer in D₂O (pD 8.4) after 0.5 to 24 hours; B) The graph of percentage of MMSa in all methyl species over time. The calculated half-life of MMSa at pD 8.4 was (34 ± 1) hour.

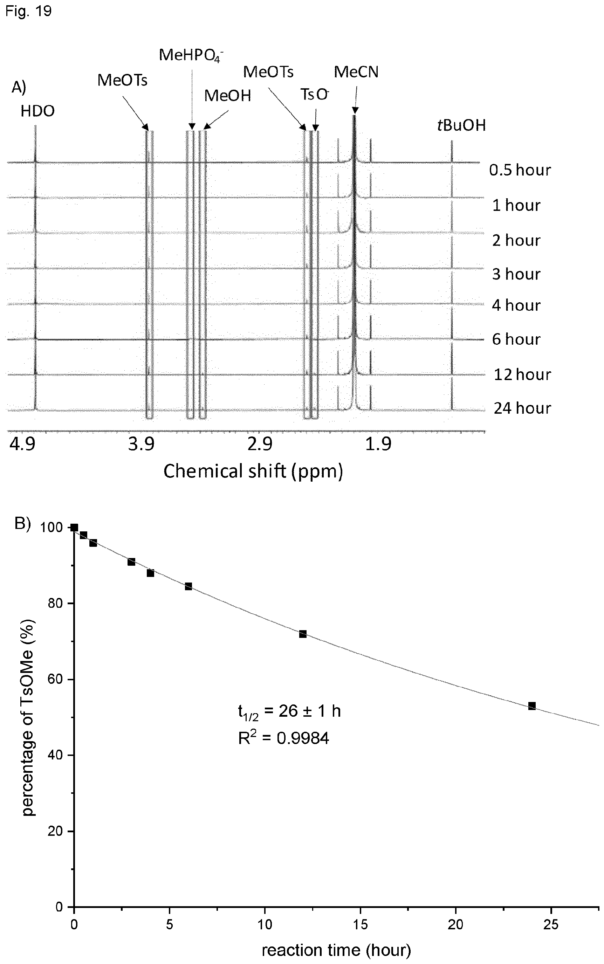

- Fig. 19: shows stability test of MeOTs in phosphate buffer. A) Stack of ¹H NMR spectra of 2 mM MeOTs in 50 mM sodium phosphate buffer in D₂O (pD 8.4) after 0.5 to 24 hours; B) The graph of percentage of MeOTs in all methyl species over time. The calculated half-life of MeOTs at pD 8.4 was (26 ± 1) hour.

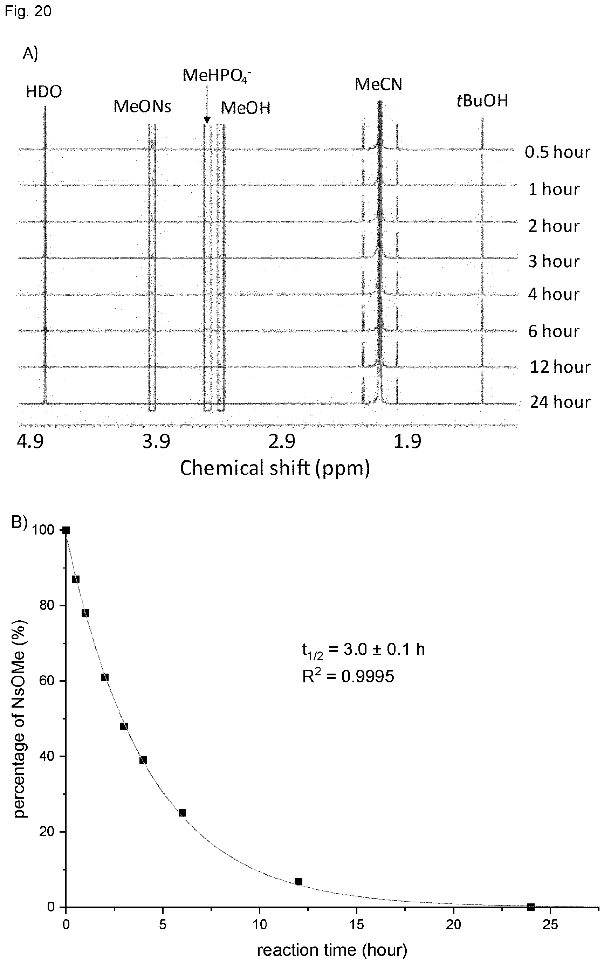

- Fig. 20: shows stability test of MeONs in phosphate buffer. A) Stack of ¹H NMR spectra of 2 mM MeONs in 50 mM sodium phosphate buffer in D₂O (pD 8.4) after 0.5 to 24 hours; B) The graph of percentage of MeONs in all methyl species over time. The calculated half-life of MeONs at pD 8.4 was (3.0 ± 0.1) hour.

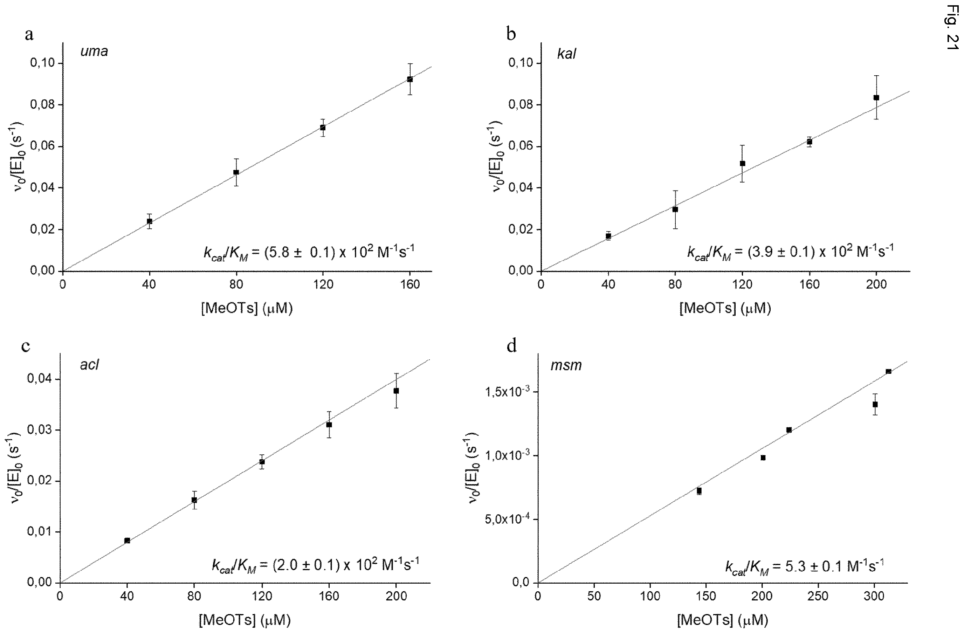

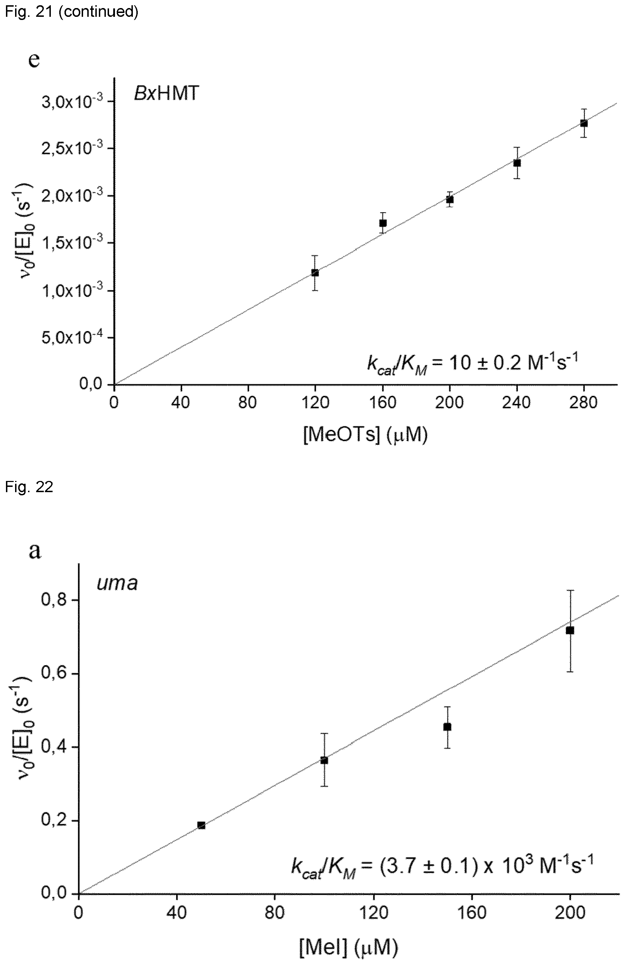

- Fig. 21: shows the catalytic efficiencies of selected TPMTs to produce SAM from SAH and MeOTs were estimated from the slope of linear fits of initial rates measured at different concentrations of MeOTs and saturating concentrations of SAH. 200 µL reactions containing 50 mM sodium phosphate buffer (pH 8.0), 1 µM enzyme (*uma, kal, acl*) or 10 µM (*msm,* BxHMT), 1 mM of SAH and different concentration of MeOTs were incubated at 25 °C. 20 µL aliquots of these reactions were quenched by adding to 20 µL of 1 % trifluoroacetic acid (TFA) at different time points. Concentrations of SAM were monitored by cation-exchange HPLC. Stock solutions of MeOTs were prepared in acetonitrile and the volume of acetonitrile added to reactions was 2.5 % of total volume.

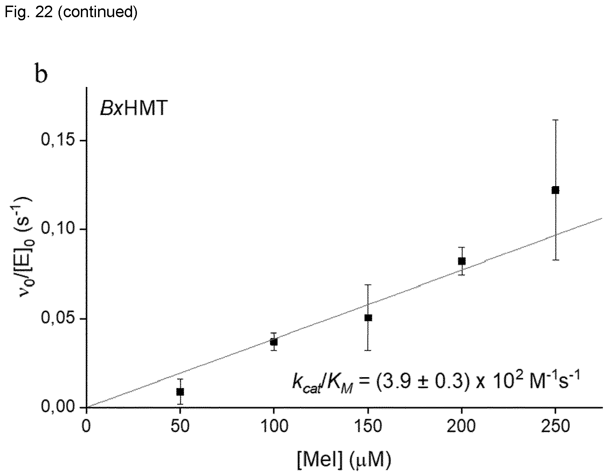

- Fig. 22: shows the catalytic efficiencies of *uma* and *Bx*HMT to produce SAM from SAH and Mel were estimated from the slope of linear fits of initial rates measured at different concentrations of Mel and saturating concentrations of SAH. 200 µL reactions containing 50 mM sodium phosphate buffer (pH 8.0), 0.1 µM *uma* or *Bx*HMT, 1 mM of SAH and different concentration of Mel from 50 µM to 200 µM were incubated at 25 °C. Sample and data analysis followed the same protocol as described above (Figure 21).

### Examples

### Example 1: Dimethylsulfate as a substrate for BxHMT

Initially, the inventors explored the idea that Mel could be generated *in situ* by reacting dimethyl sulfate (DMSa) with sodium iodide (Nal). Reactions containing HMT from *Paraburkholderia xenovorans* (BxHMT), 1 mM SAH, 2 mM DMSa, 0.1, 1.0 or 10 mM Nal in 50 mM phosphate buffer pH 8 indeed converted 60 - 70 % SAH to SAM within two hours (Figure 11). Surprisingly, *Bx*HMT produced SAM with the same efficiency even in the absence Nal, suggesting that DMSa, rather than Mel was the direct substrate of the enzyme. By contrast, efficient production of SAM by the HMT from *Arabidopsis thaliana* (*At*HMT) required the presence of 10 mM Nal (Figure 11), suggesting that DMSa is not a direct substrate of this enzyme. Less than 1 % SAH was methylated in the absence of *Bx*HMT or *At*HMT.

### Example 2: Structural differences between bacterial and plant HMT

To explore as to why the bacterial enzyme accepts DMSa as substrate whereas the plant homolog cannot, the inventors determined the crystal structure of *Bx*HMT in complex with SAH. With a resolution of 1.8 Å the observed electron density map allowed the placement of a continuous polypeptide chain from Phe14 to Ala209. Residual electron density in the active site could be modelled as SAH with high confidence. A chloride anion is positioned next to the thioether of SAH indicating the location where the halide would bind to accept a methyl group from SAM. The equivalent position in the *At*HMT structure is occupied by a crystallographic water. Despite low sequence identity of 28 %, *Bx*HMT and *At*HMT (PDB code: 3LCC) adopt highly similar overall structures (r.m.s.d. over 162 residues: 1.958 Å). The most notable difference between the two enzymes maps to their N-termini. Residues 18 - 35 in *At*HMT fold to an alpha-helix that covers the halide binding site next to SAH/SAM (Figure 2). The N-terminus of *Bx*HMT is shorter and does not fold to a helix in the crystal. Consequently, the halide binding site remains connected to the solvent. Possibly, the lid over the active site of *At*HMT restricts the type and size of guests that may bind in this pocket.

A second conspicuous difference occurs in the active site close to the thioether function of SAH. The halide binding site in *Bx*HMT and *At*HMT is formed by 10 apolar side chains (Figure 2), 7 of which are identical in both enzymes (*Bx*HMT numbering: Trp23, Pro33, Trp34, Cys125, Ala126, Pro162, Pro163) and adopt similar positions and conformations. The positions of Ala122, Val193 and Phe194 in *Bx*HMT are occupied by Val140, Thr213 and Arg214 in *At*HMT. A structural model illustrates that *Bx*HMT can accommodate DMSa in a position that would allow methyl transfer to SAH (Figure 3). In the analogous complex of *At*HMT, DMSa clashes with the side chain of Val140 (Figure 3). Hence, steric interaction could prevent turnover of DMSa by the plant enzyme. However, mutating Val140 to Ala (*At*HMT_{V140A}) did not increase activity with DMSa, but rather diminished the activity for Mel by 7-fold (Figure 12). Similarly, a previous study showed that mutating Val140 to Thr reduced the catalytic efficiency for Mel by 6-fold. Apparently, the hydrophobic isopropyl side chain of Val140 in *At*HMT is essential to immobilize Mel in juxtaposition to the sulfur atom of SAH in order to facilitate methyl transfer. This mechanism for substrate activation, combined with the N-terminal helix that closes over the active site suggests that volume and polarity of the halide binding cavity are the key parameters that determine the different activities of *At*HMT and *Bx*HMT with DMSa.

### Example 3: Prospecting the thiopurine methyltransferase family

The inventors are not aware of any reports on SAM-dependent methyltransferases that transfer methyl groups from or to sulfates or sulfonates.^{[18]} Nevertheless, based on the promiscuous activity of *Bx*HMT the inventors suspected that related enzymes may show even higher specific activity with such unnatural methyl donors. To test this idea, the inventors assayed a panel of 13 distantly related TPMT homologs for the ability to produce SAM from SAH using DMSa, methyl methanesulfonate (MMSa), methyl toluenesulfonate (MeOTs) or methyl 4-nitrobenzenesulfonate (MeONs) as methyl donor. The proteins were produced in E. *coli* and purified following standard procedures . The activity of the enzymes was assayed in reactions containing 1 mM SAH, 50 mM phosphate buffer pH 8.0 and 2 mM of either methyl donor. The concentration of SAM after different incubation times (0.5- 24 h) was quantified by HPLC. The data collected by this approach lead to the following conclusions (Figure 4 and Figures 13-15): First, the efficiencies by which enzymes accept DMSa vary by two orders of magnitude. For example, the enzymes from *Aspergillus clavatus* (*acl*) and *Dichomitus squalens (dsq)* catalyze complete conversion of 1 mM SAH to SAM within 0.5 h (rate > 3 turnover min⁻¹), whereas the activity of *bra, mac, bma, dba, nba, msm* barely exceed the background of uncatalyzed methylation (rate < 0.01 min⁻¹). Secondly, for each electrophile the inventors found a different distribution of activities among the screened enzymes. Unlike DMSa, MMSa is consumed most efficiently by the enzyme from *Vibri parhaemolyticus* (*vpa*)*,* whereas *acl* and the enzymes from *Ustilago maydis* (*uma*) and *Kordia algicida* (*kal*) were the fastest consumers of MeOTs and MeONs. The somewhat unpredictable distribution of activities across the screened panel matches the expectation for catalytic promiscuity. In a nutshell, enzymes that have evolved to catalyze specific physiological reactions may have acquired - by chance - considerable activities for reactions they were never optimized for, while others - from the same enzyme family and with the same physiological function - may have not.

A sequence alignment shows that some of the most active enzymes in this screen (*uma, acl, dsq, vpa*) share the same seven conserved residues in the halide binding site as *Bx*HMT and *At*HMT. Interestingly, all homologs with notable activities have a Thr instead of Ala at position 122 (*Bx*HMT numbering), suggesting that a beta-hydroxide side chain might support the activation of sulfate or sulfonate substrates.

The four methyl donors included in this screen are characterized by significantly different reactivities. Under conditions used to assay enzyme activities DMSa decays via hydrolysis and phosphorolysis with a half-life of (0.95 ± 0.07) h (pH 8.4, Figure 17). MMSa, MeOTs and MeONs decay through the same processes with half-lives of (28 ± 4) h, (21 ± 3) h and (2.9 h ± 0.1) h (Figures 18-20). Because of the relative stability of MeOTs, and the significant efficiency by which certain enzymes catalyze its demethylation, the inventors focused their subsequent efforts on this reagent. The inventors measured the initial rate of SAM production by several enzymes in the presence of saturating SAH and varying concentrations of MeOTs. Remarkably, the enzymes *uma, kal* and *acl* mediate methyl transfer from MeOTs to SAH with catalytic efficiencies of 580, 390 and 200 M⁻¹s⁻¹ (Table 1, entries 1 - 3). These values are similar to the efficiencies by which *Bx*HMT consumes Mel (Table 1, entry 5), and within an order of magnitude of the reported efficiency of *At*HMT. These parameters suggest that the combination of MeOTs and *uma* could allow regeneration of SAM from SAH with the same efficiency as the combination of Mel and *Bx*HMT used in previous studies.

**Table 1. Catalytic efficiencies for TPMT-catalyzed methylation.**

| | | **MeOTs** ^{a} | ***Mel*** ^{b} | |
|---|---|---|---|---|
| **entry** | **enzyme** | ***k*_{cat}/*K*_{M} (M⁻¹s⁻¹)** | ***k*_{cat}/*K*_{M} (M⁻¹s⁻¹)** | |
| 1 | *uma* | (5.8 ± 0.1) × 10² | (3.7 ± 0.1) × 10³ | |
| 2 | *kal* | (3.9 ± 0.1) × 10² | - | |
| 3 | *acl* | (2.0 ± 0.1) × 10² | - | |
| 4 | *msm* | 5.3 ± 0.3 | - | |
| 5 | *Bx*HMT | 10 ± 0.7 | (3.9 ± 0.3) × 10² | |

| | | | | |
|---|---|---|---|---|
| ^{a} Catalytic efficiencies were determined under the following conditions: 1 mM SAH, 50 mM phosphate buffer pH 8.0, 25°C, [MeOTs] ranging from 40 to 300 µM, 1 µM enzyme (*uma, kal, acl*) or 10 µM (*Bx*HMT, *msm*)*.* [SAM] was quantified by HPLC (Figure 21). ^{b} [Mel] ranging from 40 to 250 µM, 0.1 µM of *uma* or *Bx*HMT (Figure 22). | | | | |

To test this proposition, the inventors combined *uma* with several secondary MTs and examined the efficiency by which these cascades can transfer methyl groups from MeOTs to specific C-, N- or O-nucleophiles. In a reaction containing 40 µM SAH, 20 µM *uma,* 6 mM MeOTs in 50 mM phosphate buffer pH 7.0 at 25°C 40 µM putrescine *N*-methyltransferase (PMT, EC 2.1.1.53) methylated > 87 % of 2 mM putrescine (**1**, Figure 5) to *N,N'-*dimethylputrescine (**2**) within 24 h. ¹H NMR suggested that 11 % of the product reacted with an additional equivalent of MeOTs to form *N,N,N'*-trimethylputrescine (**3**). In the absence of enzymes, only 1 % of putrescine reacts with MeOTs, confirming that the secondary amine of **2** is more vulnerable to uncatalyzed methylation than the primary amine.

In a similar reaction the *trans*-aconitate 3-methyltransferase (TAMT, EC 2.1.1.145) converted 3-isopropylmalate (**4**) and *trans*-aconitate (**6**) quantitatively (> 98 %) to the respective methyl esters (**5**). The soluble form of human catechol O-methyltransferase (COMT, EC 2.1.1.6) methylated dopamine (**8**), 3,4-dihydroxyphenylacetic acid (**11**), caffeic acid (**14**) to the corresponding 3- and 4-methoxy derivatives (**9** + **10**; **12** + **13**, **15** + **16**) in ratios that are consistent with the relaxed regio-specificity of the soluble form of COMT. Importantly, all three enzyme-catalyzed reactions completely consumed their substrates, whereas no uncatalyzed reactions were observed between catechols and MeOTs. Finally, the α-keto acid methyltranferase SgvM (EC 2.1.1.281) consumed α-ketoisovaleric acid (**17**) to produce **18** with 96 % yield. SgvM is a comparatively slow enzyme (turnover frequency = 0.01 s⁻¹). Hence the reaction required 48 h to reach completion, and additional doses of 2 mM MeOTs after 12, 24 and 36 h to replace the methyl donor lost by hydrolysis.

### Example 4: 5-methylthioadenosine as alternative methyl carrier

S-adenosylmethionine is not a stable compound. Epimerization at the sulfonium center, hydrolytic loss of the nucleobase or disintegration into homoserine lactone and 5-methylthioadenosine (MTA) are the most important pathways for degradation. This inherent instability has inspired us to examine derivatives of S-adenosylhomocysteine that may be more stable towards these processes. As a first step the inventors have examined the ability of members of the thiopurine methyltransferase protein family (MTs) to methylate MTA using methyltoluolsulfonate (MeTOs) as methyl donor. The inventors found that numerous MTs can methylate 1 mM MTA to S,S-dimethyl thioadenosine (DMTA) within four hours or less (Figure 6).

### Example 5: Ethylene sulfate as a reagent for enzyme-catalyzed alkylation of S-adenosylhomocysteine

Ethylene sulfate (C₂H₄SO₄) is a two carbon-unit cyclic sulfate. The inventors also used ethylene sulfate to perform SAH alkylation (Figure 7). A new peak with retention time at 15.7 minutes could be detected by HPLC in the reactions with about half number of TPMTs, although the new peak has not been identified as the desired product. If the new peak is the target product, especially for TPMT kal, 37% of SAH was converted to SAM analog after 4-hour incubation. For TPMT CAK and ath_V140A, they also got over 20% yield of SAM analog. In my opinion, the inactivation of TPMT during the reaction was not the reason of relatively low yield of SAM analog. According to the inventors' previous work, ethylene sulfate was very easy to hydrolyze in phosphate buffer (half-life of only 11 minutes at pH 8.0). During the reaction, most of ethylene sulfate hydrolyzed so that there was not enough ethylene sulfate to alkylate SAH.

To improve the yield of enzyme-catalyzed alkylation with ethylene sulfate and to identify the product, the inventors alkylated 1 mM of SAH by adding 1 mM of ethylene sulfate every half an hour and monitored the product concentration by ion-exchange HPLC (Figure 8a). After 3-hour incubation, the yield of SAM analog reached to 60%. The reaction mixture was also analyzed by HR-ESI-MS to identify the product (Figure 8b). The m/z of 411.1442 was detected which seemed to be the mass of S-ethenylated SAH (C₁₆H₂₃N₆O₅S⁺). S-ethenylated SAH could formed during the reaction or MS analysis by elimination of a SO₄ group.

### Example 6: Dimethyl sulfite as a reagent for enzyme-catalyzed methylation of S-adenosylhomocysteine

Dimethyl sulfite (Me₂SO₃) is used as an additive in some polymers to prevent oxidation. It is much less toxic than dimethyl sulfate and can be considered as a potential methyl group donor. Herein, the inventors performed SAH methylation with dimethyl sulfite (Figure 9). To the inventors' surprise, TPMT acl gave 14% yield of SAM after 20-hour incubation. TPMT uma and TBU could also convert SAH to SAM with 8% and 7% yield, respectively.

The inventors hypothesized that the reason of dimethyl sulfite enzymatic methylating SAH to SAM was that: a) dimethyl sulfite was indeed a proper substrate of TPMT-catalyzed SAH methylation; b) dimethyl sulfite was slowly oxidized to dimethyl sulfate by air and then low concentration of dimethyl sulfate methylated SAH to SAM with a relatively steady rate. To prove this hypothesis, the inventors used these three TPMTs to performed SAH methylation in degassed buffer solution in glovebox (Figure 10). In this case, the O₂ level was very low so that dimethyl sulfite would not be oxidized to dimethyl sulfate. From the HPLC results, the inventors found that there was no significant difference of the SAM yield between performing under air and in glovebox. This indicated that dimethyl sulfite itself was a proper substrate of TPMT-catalyzed SAH methylation and could methylate SAH directly.

### Example 7: Sequences

SEQ ID NO: 1 of the invention relates to the protein sequence stated below

>ABE28953.1 Conserved hypothetical protein [Paraburkholderia xenovorans LB400]

MSDPTQPAVPDFETRDPNSPAFXDERFERRFTPXDQAGVPAAFQSFAARHSGAAVLIPXCX
SAYEAVWLAGQGNPVRAIXFSPAAVAAAHEQLGAQHAQLVEQADFFTYEPPFTPAWIYERX
FLCXLPLARRADYAHRMADLLPGGALLAGFFFLGATPKGPXFGIERAELDALLTPYFDLIEDE
AVHDSIAVFAGRERWLTWRRRA (SEQ ID NO: 1),

wherein X at position 23 can be Trp, Tyr or Phe; X at position 34 can be Trp, Tyr or Phe; X at position 59 can be Gly; X at position 61 can be Gly; X at position 80 can be Asp or Glu; X at position 122 can be Ala, Ser or Thr; X at position 126 can be Gly, Ala, Ser or Thr and X at position 163 can be Ala, Pro, Ser.

Additionally, the following sequences are incorporated in here by reference of their UNIPROT Identifier (see Table 2).

**Table 2. Overview of thiopurine methyltransferases (TPMTs) and other sequences used in this document.^{a,b}**

| **TPMT** | **Organism** | **UniProt Identifier** |
|---|---|---|
| *Bx*HMT | *Paraburkholderia xenovorans* | Q145N6 |
| *At*HMT | *Arabidopsis thaliana* | Q0WP12 |
| *At*HMT_{V140A} | *Arabidopsis thaliana* | - |
| bra | *Brassica rapa chinensis* | A1YSK2 |
| kal | *Kordia algicida* | A9DNE6 |
| mac | *Methanosarcina Acetivorans* | Q8TNZ0 |
| uma | *Ustilago maydis* | A0A0D1DT00 |
| vpa | *Vibri parhaemolyticus* | Q87H19 |
| acl | *Aspergillus clavatus* | A1CIS5 |
| bma | *Batis maritima* | Q7ZSZ7 |
| sel | *Synechococcus elongates* | Q31S13 |
| dba | *Dehalococcoidia bacterium* | A0A2E7S9H6 |
| dsq | *Dichomitus squalens* | A0A4Q9N1Z9 |
| nba | *Nitrospinae bacterium* | A0A2H0CTZ9 |
| ani | *Aspergillus niger* | A2QG34 |
| msm | *Mycolicibacterium smegmatis* | I7FMH0 |

| | **Protein** | **UniProt Identifier** |
|---|---|---|
| | putrescine *N*-methyltransferase; PMT | Q68HB1 |
| | trans-aconitate 3-methyltransferase; TAMT | P32643 |
| | catechol O-methyltransferase; COMT | P21964 |
| | SgvM | R9UTR3 |

| | | |
|---|---|---|
| ^{a} Plasmids coding for protein in entries 3-10 were obtained from S. Hammer in Bielefeld (1. K. H. Schülke et al., Angew. Chem. Int. Ed. 2021, 60, 5554-5560). ^{b} The COMT construct used by the inventors had the following mutations C33S, C69V, C95S, C157S, C173S, C188R and C191A. | | |

### Example 8: Stability tests of methyl donors in phosphate buffer

For DMSa and MMSa, they were directly dissolved in 50 mM sodium phosphate buffer in D₂O (pD 8.4) to obtain the solution with the final concentration of 4 mM methyl donor. MeOTs and MeONs were dissolved in acetonitrile and then diluted with 50 mM sodium phosphate buffer in D₂O (pD 8.4) to obtain the solution with the final concentration of 2 mM methyl donor and 5 % (v/v) acetonitrile. *tert*-Butanol was added as an inert standard to calculate the exact concentration of methyl donor. These solutions were monitored by ¹H NMR at time points between 0.5 to 24 hours to quantify the products of hydrolysis and phosphorolysis. The ¹H NMR signal peaks of all methyl species were integrated and the ratio of them was calculated. The percentage of methyl donor was plotted against time and the graph was fitted with exponential decay model to calculate the half-life of the methyl donor.

### Example 9: Conclusion

Enzyme-catalyzed methylation of SAH has emerged as a simple method for *in situ* regeneration of SAM in the context of preparative methyltransferase biocatalysis. The initial design of this method depended on Mel or MeBr as stoichiometric methyl donor. The volatility, toxicity and comparatively high cost of these reagents raised doubts as to whether this technique would be viable at scale. In search for alternative solutions the inventors observed that *Bx*HMT can also use DMSa to methylate SAH, whereas *At*HMT cannot. Structural comparison of the two distantly related enzymes revealed largely conserved halide binding sites that differ only slightly in terms of volume and polarity. A screen of 13 additional homologs of the TPMT family identified enzymes that consume DMSa, MMsa MeOTs and MeONs with even higher efficiencies - all of which share very similar sets of active site residues. Combining the fungal enzyme *uma* from *U. maydis* and MeOTs as methyl donor with secondary MTs the inventors constructed several methylation cascades that catalyze the specific methylation of N-, O- and C-nucleophiles with efficiencies that rival Mel-dependent methylation cascades.

### Example 10: Material and Methods

Unless otherwise stated, all chemicals and reagents were purchased from Sigma Aldrich and used as recieved. Antibiotics used for protein expression were purchased from PanReac AppliChem. Isopropyl β-D-thiogalactoside (IPTG) was purchased from Acros Organics. Roti^{®}garose-His/Ni beads was purchased from ROTH (Art. # 1308.2). Buffer ingredients were purchased from Acros Organics. Deuterated solvents were purchased from Cambridge Isotope Laboratories. Putrescine was purchased from Fluorochem. *trans*-Aconitic acid was purchased from Alfa.

High-performance liquid chromatography (HPLC) traces were obtained as below unless stated otherwise. 20 µL reaction samples were quenched by addition of 20 µL of 1% trifluoroacetic acid (TFA) in water. 5 µL of quenched sample was analyzed by cation-exchange HPLC. HPLC traces for enzymatic reactions were recorded on Shidmazu paired with cation-exchange LC column 150 × 4.6 mm (Luna 5 µm SCX 100 Å) under the buffer A: 20 mM H₃PO₄; B: 20 mM H₃PO₄, 1 M NaCl, pH 2, filtered with 0.22 µm MCE Membrane MF-Millipore^{™}. The HPLC gradient was programmed as follow: 0-0.5 min, 20 % B; 0.5-7.5 min, gradient from 20 to 99 % B; 7.5-10 min, 99 % B; 10-10.5 min, gradient from 99 to 20 % B; 10.5-12 min, 20 % B. Flow rate was set to 1 mL/min. The HPLC traces were exported as ASCII files and processed on OriginLab2019b.

High resolution electron spray ionization mass spectroscopy (HR-ESI-MS) spectra were obtained on a Bruker maXis 4G UHR-TOF Mass Spectrometer. All NMR spectra were recorded on a Bruker Avance Neo NMR spectrometer operating at 500 MHz proton frequency and chemical shifts were internally referenced to residual proton signals of solvents. Chemical shifts (δ) were reported in parts per million (ppm). Standard abbreviations indicating multiplicity were used as follows: s (singlet), d (doublet), t (triplet), dd (doublet of doublets), and m (multiplet). Coupling constants (*J*) were reported in Hertz (Hz).

*Plasmids.* Plasmids coding for thiopurine methyltransferases (TPMTs) bra, kal, mac, uma, vpa, acl, bma, and sel were obtained from S. Hammer in Bielefeld.¹ Among these TPMTs, genes of TPMT bra, kal, mac, uma and vpa were inserted into pET28a(+), containing a C-terminal Leu-Glu-His₆-motiv. Genes of TPMT acl, bma and sel were inserted into pBAD33 vector containing the same C-terminal Leu-Glu-His₆-motiv. Codon-optimized genes of all other TPMTs and methyltrnsferases (MTs) were purchased from BioCat GmbH as inserts in pET28a(+) plasmids as Ncol/Xho1 fragments. The expression strain SAH nucleosidase deficient cell *E. coli* Δmtn (DE3) was constructed in a previous study (C. Liao, F. P. Seebeck, Nature Catalysis 2019, 2, 696-701).

*Transformation.* The expression plasmids (pET28a(+) or pBAD33) were transformed into *E*. *coli* Δmtn (DE3) cell by electroporation using a BIO-RAD GENE PULSER^{®} II. The voltage was set to 2 kV, resulting in a time constant of around 5 msec. Capacitance was set at 30 µF. Immediately after applying the electric pulse, cells were suspended in 1 mL of 37 °C Lysogeny Broth (LB). The cell culture was incubated at 37 °C for a minimum of 45 minutes before plating on LB-Agar plates containing antibiotics (pET28a(+): kanamycin, pBAD33: chloramphenicol).

*Cell culture.* Cells containing the plasmid were collected from LB-AGAR plates with antibiotics and used to inoculate LB medium. After incubation at 37 °C overnight, every liter of sterilized Terrific Broth (TB) with antibiotics (pET28a(+): 50 mg/L kanamycin, pBAD33: 35 mg/L chloramphenicol) was inoculated by 5 mL of pre-culture. The 1 L of cultures were incubated in 3 L shaking flask at 37 °C (180 rpm) until OD600 reached 0.6 and then the cultures were allowed to cool down to 18 °C by changing the set temperature of the shaker. The cultures were supplemented with 100 µM Isopropyl β-D-thiogalactoside (IPTG) and incubated further at 18°C for 20 h. Cells were harvested by centrifugation at 17,568 × g for 25 minutes at 4 °C and stored at -20 °C for a minimum of 2 hours before purification.

*Protein purification.* For purification, frozen cell pellets were suspended in 4 mL per gram of wet cells (ml/g_{cww}) of lysis buffer (50 mM sodium phosphate, 300 mM NaCl, pH 8.0). The suspension was disrupted at 4 °C by sonication for 3 × 60 seconds with a Branson sonifier 450 (output control 5, 50% duty cycle). The suspension was homogenized at 4 °C by EmulsiFlex at 1000 bar applied with 4 bar pressure of N₂. The cell-free lysates were cleared by centrifugation at 47,850 × g for 45 minutes at 4 °C. Every 25 mL of clear lysate was supplemented with 1 ml of Ni^{ll} NTA agarose slurry and incubated at 4 °C for 20 minutes. Subsequently, this suspension was loaded onto a column (10 mL polypropylene column, Thermo Scientific) with a matching polyethylene frit at the bottom. The agarose beads were washed with 10 mL cold lysis buffer containing 10 mM and 20 mM imidazole respectively. The protein was eluted in cold lysis buffer solution containing 250 mM imidazole. The absorbance at 280 nm of the eluted fractions was measured on NanoDrop 2000 Spectrophotometer (Thermo Scientific). Fractions with Abs₂₈₀ₙₘ > 0.2 were combined and dialyzed in a cellulose bag (MW 12-14 kDa cutoff, 23 µm thick, art.# E684.1, purchased from ZelluTransROTH^{®}) against dialysis buffer (50 mM sodium phosphate, pH 8.0). The final protein concentration was determined by measuring Abs₂₈₀ₙₘ against the dialysis buffer. The protein solution was aliquoted, frozen in liquid nitrogen and stored at -80 °C. The homogeneity of the purified enzymes was assessed by sodium dodecyl sulfate polyacrylamide gel electrophoresis (SDS-PAGE) under denaturing conditions. The gels were stained with Coomassie brilliant blue.

*Crystallization and diffraction data collection.* Sitting drop vapor diffusion method was applied for the crystallization of *Bx*HMT. The protein sample *Bx*HMT (15 mg/mL, 0.6 mM) as well as the co-substrate SAH (1.0 mM final conc.) were present in Tris-HCI (pH 8.0, 20 mM), NaCl (200 mM) buffer. Prior to crystallization the protein substrate mixture was incubated for 90 min. On a 96-well 3-drop plate (Swissci) 0.1 µL reservoir solution of crystallization screen was combined with 0.2 µL protein solution. Sealed plates were stored at 20 °C for reservoir / protein equilibration and formation of crystals was observed after one to three days in a drop containing 18 % PEG 6000, Tris-HCI (0.09 M, pH 8.0), CaCl₂ (0.18 M) and LDAO (0.5 %, n-dodecyl-N,N-dimethylamine-N-oxide). Crystals were cryo-protected with ethylene glycol (20 %), looped and flash frozen in liquid nitrogen approximately 3 weeks past crystallization. Diffraction data were collected at X06SA (PXI) beamline of Swiss Light Source (Paul Scherrer Institute, Villigen, Switzerland) equipped with an Eiger-16M detector (Dectris). High resolution data sets were collected at an X-ray energy of 12398 eV (λ= 1.000 Å) at the temperature 100 K.

*Data processing and refinement.* Data were indexed and integrated with XDS (W. Kabsch, Acta Crystallogr. D Biol. Crystallogr. 2010, 66 (2), 125-132) and scaled and merged with Aimless (P. R. Evans, G. N. Murshudov, Acta Crystallogr. D Biol. Crystallogr. 2013, 69 (7), 1204-1214) from the CCP4 program suite (P. Evans, Acta Crystallogr. D Biol. Crystallogr. 2006, 62 (1), 72-82; M. D. Winn et al., Acta Crystallogr. D Biol. Crystallogr. 2011, 67 (4), 235-242). *Bx*HMT crystals grew into orthorhombic space group C 2 2 2₁ with unit cell dimensions of a = 63.9 Å, b = 90.8 Å, c = 74.9 Å, α = 90°, β = 90°, γ = 90°. A single protein monomer was contained per unit cell. Given the good diffraction quality the data were scaled to a high-resolution limit of 1.8 Å. The crystal structure of *At*HMT (PDB ID: 3LCC) was used as search model for molecular replacement using Phenix Phaser (A. J. McCoy et al., Appl. Crystallogr. 2007, 40 (4), 658-674). Iterative cycles of manual and automated model building into the readily interpretable electron density map and refinement using COOT (P. Emsley et al., Acta Crystallogr. D Biol. Crystallogr. 2010, 66 (4), 486-501; P. Emsley, K. Cowtan, Acta Crystallogr. D Biol. Crystallogr. 2004, 60 (12), 2126-2132) and phenix.refine (P. V. Afonine et al., Acta Crystallogr. D Biol. Crystallogr. 2012, 68 (4), 352-367; T. C. Terwilliger, Acta Crystallogr. D Biol. Crystallogr. 2004, 60 (12), 2144-2149; V. B. Chen et al., Acta Crystallogr. D Biol. Crystallogr. 2010, 66 (1), 12-21). *Bx*HMT polypeptide chain was modeled into continuous electron density for residue 14 - 206. Aside of the protein chain the asymmetric unit cell contained additional electron density that could have readily been modeled by adding the co-substrate SAH molecule.

### Cited prior art documents:

All scientific publications and patent documents cited in the present specification are incorporated by reference herein.
K. H. Schülke, F. Ospina, K. Hörnschemeyer, S. Gergel, S. C. Hammer, ChemBioChem 2022, 23, e202100632.
C. Liao, F. P. Seebeck, Nature Catalysis 2019, 2, 696-701.
P. R. Evans, G. N. Murshudov, Acta Crystallogr. D Biol. Crystallogr. 2013, 69 (7), 1204-1214 P. Evans, Acta Crystallogr. D Biol. Crystallogr. 2006, 62 (1), 72-82.
M. D. Winn, C. C. Ballard, K. D. Cowtan, E. J. Dodson, P. Emsley, P. R. Evans, R. M. Keegan, E. B. Krissinel, A. G. Leslie, A. McCoy, S. J. McNicholas, G. N. Murshudov, N. S. Pannu, E. A. Potterton, H. R. Powell, R. J. Read, A. Vagin, K. S. Wilson, Acta Crystallogr. D Biol. Crystallogr. 2011, 67 (4), 235-242.
P. Emsley, B. Lohkamp, W. G. Scott, K. Cowtan, Acta Crystallogr. D Biol. Crystallogr. 2010, 66 (4), 486-501.
P. Emsley, K. Cowtan, Acta Crystallogr. D Biol. Crystallogr. 2004, 60 (12), 2126-2132.
W. Kabsch, Acta Crystallogr. D Biol. Crystallogr. 2010, 66 (2), 125-132.
P. V. Afonine, R. W. Grosse-Kunstleve, N. Echols, J. J. Headd, N. W. Moriarty, M. Mustyakimov, T. C. Terwilliger, A. Urzhumtsev, P. H. Zwart, P. D. Adams, Acta Crystallogr. D Biol. Crystallogr. 2012, 68 (4), 352-367.
T. C. Terwilliger, Acta Crystallogr. D Biol. Crystallogr. 2004, 60 (12), 2144-2149.
V. B. Chen, W. B. Arendall, J. J. Headd, D. A. Keedy, R. M. Immormino, G. J. Kapral, L. W. Murray, J. S. Richardson, D. C. Richardson, Acta Crystallogr. D Biol. Crystallogr. 2010, 66 (1), 12-21.
A. J. McCoy, R. W. Grosse-Kunstleve, P. D. Adams, M. D. Winn, L. C. Storoni, R. J. Read, J. Appl. Crystallogr. 2007, 40 (4), 658-674.

## Claims

1. A method for production of an alkylated carrier compound comprising the steps:
a. providing a dealkylated carrier compound CC;
b. in an alkylation step applying:
i. T-X, wherein an alkyl-group donor X is bound to an alkyl-group T ; and
ii. a methyltransferase transferring an alkyl group T from X to CC; yielding an alkylated carrier compound T-CC;
**characterized in that** the alkyl-group donor X is sulfur-, or selenium-, or oxygen-based.

2. The method according to claim 1, wherein T is of the formula -CH₂-R⁰ or -CHF-R⁰ with R⁰ being selected from H, CN, F, Cl or an unsubstituted or substituted moiety MM being unfunctionalized or functionalized, wherein MM is selected from the group comprising alkyl, aryl, heteroaryl, alkyl-aryl, alkyl-heteroaryl;
wherein functionalized MM comprises an alkene, an alkyne, an ether, a thioether, an ester, disulfide, and/or a thioester moiety,
wherein substituted MM is substituted with one or two or three moieties selected from the group comprising halogen, OH, NH₂, N₃, CN, COOH, NO₂, CONH₂, SO₃⁻; SO₂NH₂, epoxide;
particularly MM is selected from the group comprising alkyl, aryl, alkyl-aryl,
more particularly MM is alkyl.

3. The method according to any one of the preceding claims, wherein T-X is selected from the group comprising 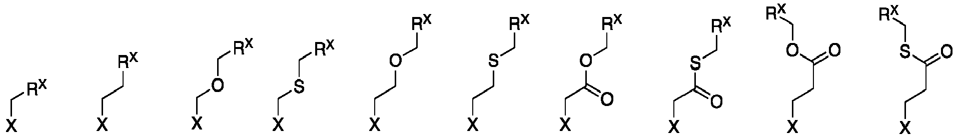
particularly T-X is selected from the group comprising 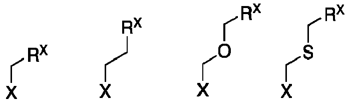
with R^{X} being selected from the group comprising H, F, OCH₃, OCH₂CH₃, CN, - COOH, NO₂, CF₃, CHF₂, CH₂F, CH₂Cl, CH₂Br, CONH₂, CH₂CO₂R^{X1},CH₃, (CH₂)ₘCH₃, CH(CH₃)₂, C₂H, CH₂C₂H, CH=CH₂, CH₂CH=CH₂, unsubstituted or R^{X2}-substituted O-aryl or aryl; with m being an integer selected from the group comprising 1 - 14; with R^{X1} being selected from the same group as R^{X} excluding CH₂CO₂R^{X1}; with each R^{X2} being independently selected from CH₃, (CH₂)ₘCH₃, CH(CH₃)₂, C(CH₃)₃, F, Cl, Br, I, OH, OR, NH₂, NHCH₃, NO₂, CN, CO₂, CF₃, CHF₂, CH₂F, CH₂Cl, CH₂Br, CONH₂, CH₂CO₂CH₃, SO₃⁻;
more particularly T-X is selected from the group comprising 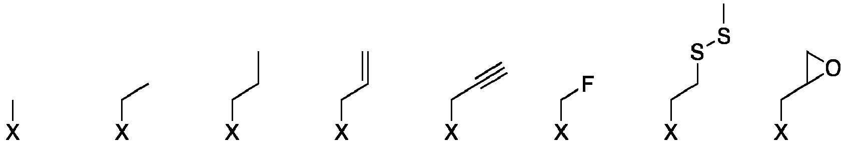 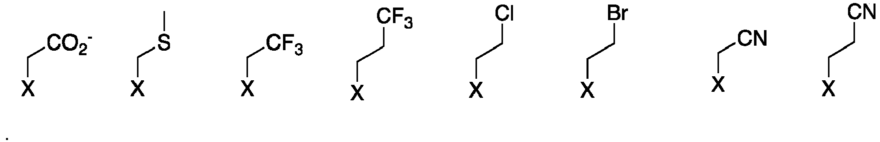
most particularly T is methyl.

4. The method according to any one of the preceding claims, wherein CC is of the general formula: Z-P-Rib-Nuc,
wherein
- Z is of the formula -CH₂-R^{Z} with R^{Z} being selected from H, or an unsubstituted or substituted moiety NN being unfunctionalized or functionalized, wherein NN is selected from the group comprising alkyl, aryl, heteroaryl, alkyl-aryl, alkyl-heteroaryl,
wherein functionalized NN comprises an alkene, an alkyne, an ether, a thioether, an ester, and/or a thioester moiety,
wherein substituted NN is substituted with one or two or three moieties selected from the group comprising halogen, CN, COOH, NO₂, NH₃, CONH₂, SO₃H;
particularly NN is alkyl;
- P is a sulfur atom or a selenium atom;
- Rib is selected from the group comprising ribose, deoxyribose, 2-O-methyl ribose, carbaribose, 4-thioribose, particularly Rib is ribose;
- Nuc is a nucleobase or a nucleobase derivative;
wherein P is connected to the 5' C atom of Rib, and Nuc is connected to the O atom of the 1' C atom of Rib.

5. The method according to any one of the preceding claims, wherein CC is selected from the group comprising 5'-deoxy-5'-alkylthionucleoside and 5'-deoxy-5'-alkylselenonucleoside, with alkyl being selected from the group comprising methyl, fluormethyl, ethyl, propyl, 2-carboxy-2-aminoethyl, 3-aminopropyl, 3-hydroxypropyl, 3-carboxypropyl, 4-carboxybutyl, 4-aminobutyl, 4-hydroxybutyl, 3-carboxy-3-aminopropyl, 4-carboxy-4-aminobutyl,
particularly with alkyl being selected from the group comprising 2-carboxy-2-aminoethyl, 3-carboxy-3-aminopropyl;
more particularly wherein CC is selected from the group comprising S-nucleosyl-homocysteine, Se-nucleosyl-homoselenocysteine, S-inosyl-L-homocysteine, Se-inosyl-L-homoselenocysteine, S-7-deazaadenosyl-L-homocysteine, S-7-deazaadenosyl-L-homoselenocysteine, S-formycinyl-L-homocysteine, Se-formycinyl-L-homoselenocysteine, S- aristeromycyl-L-homocysteine, Se- aristeromycyl-L-homoselenocysteine.

6. The method according to any one of the preceding claims 1 to 4, wherein CC is selected from the group comprising
| Systematic Name |
|---|
| (S)-2-amino-4-((((2*S*,3*S*,4*R*,5*R*)-5-(6-amino-9*H*-purin-9-yl)-3,4-dihydroxytetrahydrofuran-2-yl)methyl)thio)-1-(λ¹-oxidaneyl)butan-1-one |
| (*R*)-4-((((2*S*,3*S*,4*R*,5*R*)-5-(6-amino-9*H*-purin-9-yl)-3,4-dihydroxytetrahydrofuran-2-yl)methyl)thio)-2-hyd roxybutanoate |
| (*S*)-4-((((2*S*,3*S*,4*R*,5*R*)-5-(4-amino-7*H*-pyrrolo[2,3-*d*]pyrimidin-7-yl)-3,4-dihydroxytetrahydrofuran-2-yl)methyl)thio)-2-ammoniobutanoate |
| (*S*)-2-ammonio-4-((((2*S*,3*S*,4*R*,5*R*)-3,4-dihydroxy-5-(6-hydroxy-9*H*-purin-9-yl)tetrahydrofuran-2-yl)methyl)thio)butanoate |
| (*S*)-2-ammonio-4-((((2*S*,3*S*,4*R*,5*R*)-3,4-dihydroxy-5-(9*H*-purin-9-yl)tetrahydrofuran-2-yl)methyl)thio)butanoate |
| (*S*)-4-((((2*S*,3*S*,4*R*,5*S*)-5-((R)-7-amino-3*H*-pyrazolo[4,3-*d*]pyrimidin-3-yl)-3,4-dihydroxytetrahydrofuran-2-yl)methyl)thio)-2-ammoniobutanoate |
| (S)-4-((((1S,2R,3S,4R)-4-(6-amino-9H-purin-9-yl)-2,3-dihydroxycyclopentyl)methyl)thio)-2-ammoniobutanoate |
| (*S*)-4-((((2*S*,3*S*,4*R*,5*R*)-5-(4-amino-1*H*-imidazo[4,5-c]pyridin-1-yl)-3,4-dihydroxytetrahydrofuran-2-yl)methyl)thio)-2-ammoniobutanoate |
| (*S*)-4-((((2*S*,3*S*,5*R*)-5-(6-amino-9*H*-purin-9-yl)-3-hydroxytetrahydrofuran-2-yl)methyl)thio)-2-ammoniobutanoate |
| (*S*)-4-((((2*S*,3*S*,4*R*,5*R*)-5-(4-amino-2-oxopyrimidin-1(2*H*)-yl)-3,4-dihydroxytetrahydrofuran-2-yl)methyl)thio)-2-ammoniobutanoate |
| (*R*)-3-((((2*S*,3*S*,4*R*,5*R*)-5-(6-amino-9*H*-purin-9-yl)-3,4-dihydroxytetrahydrofuran-2-yl)methyl)thio)-2-ammoniopropanoate |
| 3-((((2*S*,3*S*,4*R*,5*R*)-5-(6-amino-9*H*-purin-9-yl)-3,4-dihydroxytetrahydrofuran-2-yl)methyl)thio)propan-1-aminium |
| (2*R*,3*R*,4*S*,5*S*)-2-(6-amino-9*H*-purin-9-yl)-5-(((3-hydroxypropyl)thio)methyl)tetrahydrofuran-3,4-diol |
| 4-((((2*S*,3*S*,4*R*,5*R*)-5-(6-amino-9*H*-purin-9-yl)-3,4-dihydroxytetrahydrofuran-2-yl)methyl)thio)butanoate |
| (*S*)-4-((((2*S*,3*S*,4*R*,5*R*)-5-(6-amino-9*H*-purin-9-yl)-3-hydroxy-4-methoxytetrahydrofuran-2-yl)methyl)thio)-2-ammoniobutanoate |
| (2*R*,3*R*,4*S*,5*S*)-2-(6-amino-9*H*-purin-9-yl)-5-((methylthio)methyl)tetrahydrofuran-3,4-diol |
| (2*R*,3*R*,4*S*,5*S*)-2-(6-amino-9*H*-purin-9-yl)-5-((ethylthio)methyl)tetrahydrofuran-3,4-diol |
| (2*R*,3*R*,4*S*,5*S*)-2-(6-amino-9*H*-purin-9-yl)-5-((propylthio)methyl)tetrahydrofuran-3,4-diol |
| 5-((((2*S*,3*S*,4*R*,5*R*)-5-(6-amino-9*H*-purin-9-yl)-3,4-dihydroxytetrahydrofuran-2-yl)methyl)thio)pentanoate |
| (*S*)-4-((((2*S*,3*S*,4*R*,5*R*)-5-(6-amino-9*H*-purin-9-yl)-3,4-dihydroxytetrahydrofuran-2-yl)methyl)selanyl)-2-ammon iobutanoate |
| (*R*)-4-((((2*S*,3*S*,4*R*,5*R*)-5-(6-amino-9*H*-purin-9-yl)-3,4-dihydroxytetrahydrofuran-2-yl)methyl)selanyl)-2-hyd roxybutanoate |
| (S)-4-((((1 S,2R,3S,4R)-4-(6-amino-9H-purin-9-yl)-2,3-dihydroxycyclopentyl)methyl)selanyl)-2-ammoniobutanoate |
| (*S*)-2-ammonio-4-((((2*S*,3*S*,4*R*,5*R*)-3,4-dihydroxy-5-(6-hydroxy-9*H*-purin-9-yl)tetrahydrofuran-2-yl)methyl)selanyl)butanoate |
| (*S*)-2-ammonio-4-((((2*S*,3*S*,4*R*,5*R*)-3,4-dihydroxy-5-(9*H*-purin-9-yl)tetrahydrofuran-2-yl)methyl)selanyl)butanoate |
| (*S*)-4-((((2*S*,3*S*,4*R*,5*S*)-5-((*R*)-7-amino-3*H*-pyrazolo[4,3-*d*]pyrimidin-3-yl)-3,4-dihydroxytetrahydrofuran-2-yl)methyl)selanyl)-2-ammoniobutanoate |
| (*S*)-4-((((1*S*,2*R*,3*S*,4*R*)-4-(6-amino-9*H*-purin-9-yl)-2,3-dihydroxycyclopentyl)methyl)selanyl)-2-ammoniobutanoate |
| (*S*)-4-((((2*S*,3*S*,4*R*,5*R*)-5-(4-amino-1*H*-imidazo[4,5-*c*]pyridin-1-yl)-3,4-dihydroxytetrahydrofuran-2-yl)methyl)selanyl)-2-ammoniobutanoate |
| (*S*)-4-((((2*S*,3*S*,5*R*)-5-(6-amino-9*H*-purin-9-yl)-3-hydroxytetrahydrofuran-2-yl)methyl)selanyl)-2-ammoniobutanoate |
| (*S*)-4-((((2*S*,3*S*,4*R*,5*R*)-5-(4-amino-2-oxopyrimidin-1(2*H*)-yl)-3,4-dihydroxytetrahydrofuran-2-yl)methyl)selanyl)-2-ammoniobutanoate |
| (*R*)-3-((((2*S*,3*S*,4*R*,5*R*)-5-(6-amino-9*H*-purin-9-yl)-3,4-dihydroxytetrahydrofuran-2-yl)methyl)selanyl)-2-ammoniopropanoate |
| 3-((((2*S*,3*S*,4*R*,5*R*)-5-(6-amino-9*H*-purin-9-yl)-3,4-dihydroxytetrahydrofuran-2-yl)methyl)selanyl)propan-1-aminium |
| (2*R*,3*R*,4*S*,5*S*)-2-(6-amino-9*H*-purin-9-yl)-5-(((3-hydroxypropyl)selanyl)methyl)tetrahydrofuran-3,4-diol |
| 4-((((2*S*,3*S*,4*R*,5*R*)-5-(6-amino-9*H*-purin-9-yl)-3,4-dihydroxytetrahydrofuran-2-yl)methyl)selanyl)butanoate |
| (*S*)-4-((((2*S*,3*S*,4*R*,5*R*)-5-(6-amino-9*H*-purin-9-yl)-3-hydroxy-4-methoxytetrahydrofuran-2-yl)methyl)selanyl)-2-ammoniobutanoate |
| (2*R*,3*R*,4*S*,5*S*)-2-(6-amino-9*H*-purin-9-yl)-5-((methylselanyl)methyl)tetrahydrofuran-3,4-diol |

7. The method according to any one of the preceding claims, wherein X comprises a moiety selected from the group comprising sulfate, sulfonate, sulfite, sulfinate, thiosulfate, thiosulfonate, thiosulfinate, wherein T is attached to said moiety.

8. The method according to any one of the preceding claims, wherein T-X is selected from the group comprising 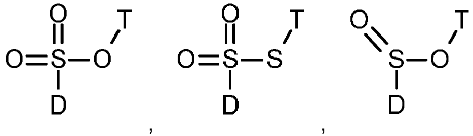 with D being selected from -R^{D1}, -OR^{D2}, -SR^{D2}, -NR^{D2}R^{D3}, with
- R^{D1} being selected from the group comprising an unsubstituted or substituted moiety LL being unfunctionalized or functionalized, wherein LL is selected from the group comprising alkyl, aryl, heteroaryl, alkyl-aryl, alkyl-heteroaryl, wherein functionalized LL comprises an alkene, an alkyne, an ether, a thioether, an ester, and/or a thioester moiety,
wherein substituted LL is substituted with one or two or three moieties selected from the group comprising halogen, CN, COOH, NO₂, NH₃, CONH₂, SO₃H;
- R^{D2} and R^{D3} being independently selected from the group comprising H or R^{D1}.

9. The method according to any one of the preceding claims, wherein T-X is selected from the group comprising
a. 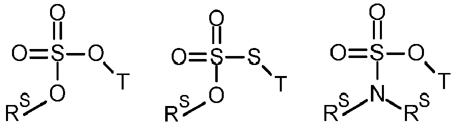 with each R^{S} being independently selected from the group comprising H, CH₃, (CH₂)ₕCH₃, CH(CH₃)₂, C(CH₃)₃, CN, CF₃, CHF₂, CH₂F, CH₂Cl, CH₂Br, CH₂NO₂, CH₂CO₂CH₃, C₆H₁₁;
b. 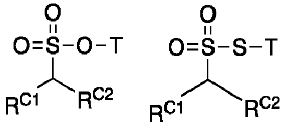 with R^{C1} and R^{C2} being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂-, CF₃, CHF₂, CH₂F, CH₂Cl, CH₂Br, CONH₂, CH₂CO₂CH₃, C₆H₁₁, SO₃⁻
c. 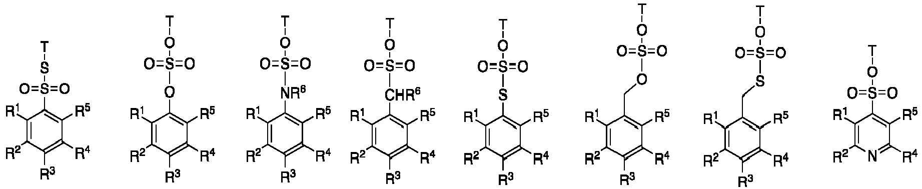 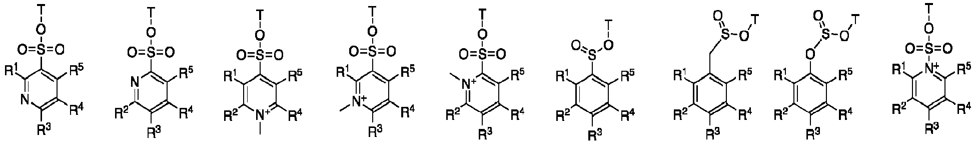
with R¹ - R⁵ being independently selected from the group comprising H, CH₃, (CH₂)ₕCH₃, CH(CH₃)₂, C(CH₃)₃, F, Cl, Br, I, OH, OR⁶, NH₂, NHR⁶, NO₂, CN, CO₂, CF₃, CHF₂, CH₂F, CH₂Cl, CH₂Br, CONH₂, CH₂CO₂CH₃, S(CH₃)₂⁺, N(CH₃)₃⁺, SO₃⁻;
in particular from H, CH₃, F, Cl, Br, OMe, CF₃, NO₂, CN, CO₂Me, CH(CH₃)₂, S(CH₃)₂⁺, N(CH₃)₃⁺, C(CH₃)₃, more particularly from H, CH₃, F, Cl, Br, OMe, CF₃, NO₂, CN,
with each R⁶ being independently selected from the group comprising H, CH_{3;}
with h being an integer selected from the group comprising 1 - 14.
particularly wherein T-X is selected from the group comprising
a. 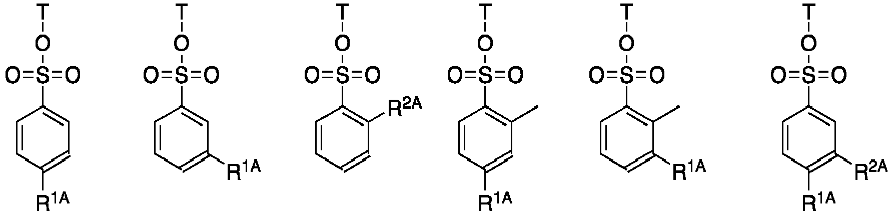 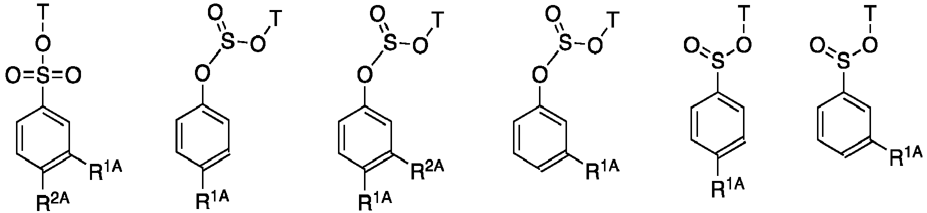
with R^{1A} being selected from the group comprising H, CH₃, F, Cl, Br, OMe, CF₃, NO₂, CN, CO₂Me, CH(CH₃)₂, S(CH₃)₂⁺, N(CH₃)₃⁺, C(CH₃)₃,
with R^{2A} being selected from the group comprising H, CH₃, F, Cl, Br, OMe, CF₃, NO₂, CN,
b. 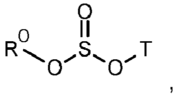 particularly: 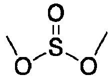 with R^{O} being selected from the group comprising H, CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃, C₆H₁₁;
c. 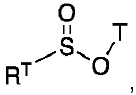 particularly 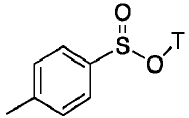
with R^{T} being selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃₁,C₆H₅, CH₂(C₆H₅), C₆H₁₁
with k being an integer selected from the group comprising 1 - 14;
particularly wherein T-X is selected from the group comprising methyl toluenesulfonate (MeOTs), methyl 4-nitrobenzenesulfonate (MeONs), dimethyl sulfate (DMSa), or methyl methanesulfonate (MMSa), dimethyl sulfite, S-methyl thiosulfate, particularly the alkyl-group donor is MeOTs.

10. The method according to any one of the preceding claims 1 to 6, wherein T-X is selected from the group comprising 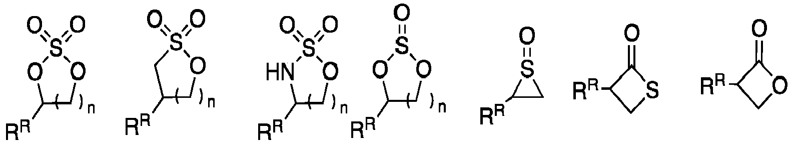 particularly T-X is selected from the group comprising 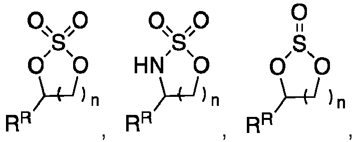 more particularly T-X is 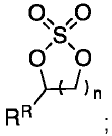 with R^{R} being selected from the group comprising H, OR, NO₂, CN, COOH, CF₃, CHF₂, CH₂F, CH₂Cl, CH₂Br, CONH₂, CH₂CO₂Me, CH₃, (CH₂)ₙCH₃, with n being an integer selected from the group comprising 1 - 14.

11. The method according to any one of the preceding claims 1 to 6, wherein T-X is selected from the group comprising 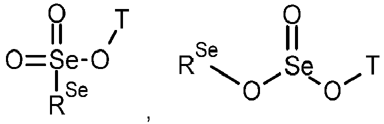 particularly wherein T-X is selected from the group comprising 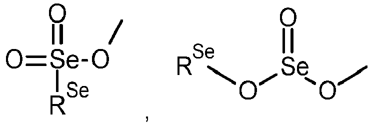 wherein
R^{Se} is selected from the group comprising CH₃, (CH₂)ₖCH₃, CH(CH₃)₂, C(CH₃)₃, CF₃, CHF₂, CH₂F, CH₂Cl, CH₂Br, CH₂NO₂, CH₂CO₂CH₃,C₆H₅, CH₂(C₆H₅), C₆H₁₁, with k being an integer selected from the group comprising 1 - 14,
particularly R^{Se} is CH₃.

12. The method according to any one of the preceding claims, wherein the methyltransferase is derived from or is an S-adenosylmethionine (SAM)-dependent methyltransferase.

13. The method according to any one of the preceding claims, wherein the methyltransferase belongs to a protein family (PF) or is derived from a protein belonging to a PF, wherein the PF is selected from the group comprising PF05724, PF03492, PF08241, particularly wherein the PF is PF05724.

14. The method according to any one of the preceding claims, wherein T comprises at least one isotope selected from a stable heavy isotope or radioisotope, particularly T is isotope-labelled methyl or isotope-labelled -CH₂F, more particularly
- T is methyl comprising ¹³C and/or ²H, or
- T is -CH₂¹⁹F or
- T is methyl comprising ¹¹C, ¹⁴C and/or ³H or
- T is -CH₂¹⁸F.

15. A method for alkylation of a substrate, the method comprising the steps:
a. providing a substrate and a dealkylated carrier compound CC;
b. in an alkylation step, alkylating CC via the method according to any one of the preceding claims yielding an alkylated carrier compound T-CC;
c. in an alkyl-substrate production step, transferring an alkyl group from T-CC to the substrate catalyzed by an N-, C-, O-, S-, or P-specific methyltransferase yielding an alkylated substrate and a dealkylated carrier compound;
wherein at least a part of the dealkylated carrier compound CC of step c is recycled to step b to regenerate the alkylated carrier compound T-CC.
